(19) **Europäisches Patentamt / European Patent Office / Office européen des brevets**

(11) **EP 4 134 092 A1**

(12) **EUROPEAN PATENT APPLICATION**
published in accordance with Art. 153(4) EPC

(43) Date of publication:
**15.02.2023 Bulletin 2023/07**

(21) Application number: **21785260.7**

(22) Date of filing: **05.04.2021**

(51) International Patent Classification (IPC):
**A61K 38/00** (1995.01)          **A61K 38/08** (1995.01)
**A61P 31/04** (2000.01)

(52) Cooperative Patent Classification (CPC):
**A61K 38/00; A61K 38/08; A61P 31/04;** Y02A 50/30

(86) International application number:
**PCT/KR2021/004232**

(87) International publication number:
**WO 2021/206397 (14.10.2021 Gazette 2021/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.04.2020 KR 20200042340**

(71) Applicant: **Camp Therapeutics Inc.**
**Seoul 08826 (KR)**

(72) Inventors:
 • **HYUN, Soon-Sil**
  **Seoul 08826 (KR)**
 • **CHOI, Yoon-Hwa**
  **Seoul 08826 (KR)**
 • **CHOO, Seol-Ah**
  **Seoul 08826 (KR)**
 • **PARK, Tae-Woo**
  **Seoul 08826 (KR)**
 • **YU, Jae-Hoon**
  **Seongnam-si, Gyeonggi-do 13531 (KR)**

(74) Representative: **Schnappauf, Georg**
**ZSP Patentanwälte PartG mbB**
**Hansastraße 32**
**80686 München (DE)**

Remarks:
The complete document including Reference Table(s) and the Sequence Listing(s) can be downloaded from the EPO website

(54) **NOVEL ANTIBACTERIAL PEPTIDE OR PEPTIDE ANALOG AND USE THEREOF**

(57) The present disclosure relates to a novel antibacterial peptide or peptide analog and a therapeutic use thereof for bacterial infections. Specifically, the present disclosure relates to a peptide or peptide analog having a structure in which an amphipathic, alpha-helical peptide composed of hydrophobic amino acids and hydrophilic amino acids is kinked, with a fatty acid bound to the N-terminus thereof, and a therapeutic use thereof for bacterial infections, especially, infections caused by Gram-negative bacteria.

**EP 4 134 092 A1**

## Description

## Technical Field

The present disclosure relates to a novel antibacterial peptide or peptide analog and a therapeutic use thereof for bacterial infections or antibacterial use. Specifically, the present disclosure relates to a peptide or peptide analog having a structure in which an amphipathic, alpha-helical peptide composed of hydrophobic amino acids and hydrophilic amino acids is kinked, with a fatty acid bound to the N-terminus thereof, and a therapeutic use thereof for bacterial infections, especially, infections caused by Gram-negative bacteria.

## Background Art

There are a large number of therapeutic agents for Gram-positive bacteria; however, there is no therapeutic agent for Gram-negative bacteria. It is known that this is because antibiotic candidates do not pass through the outer membrane of Gram - negative bacteria.

In Gram-negative bacteria, the outer membrane starting with a lipopolysaccharide (LPS) layer has both hydrophilicity and hydrophobicity due to the LPS layer. Thus, most low-molecular-weight drugs are unable to pass through the membrane freely. It is known that most of the antibiotics, which are effective against Gram-positive bacteria but not against Gram-negative bacteria, do not pass through the outer membrane. Representative drugs include linezolid, cloxacillin, and the like.

Attempts have been made to use antibacterial agents for Gram-positive bacteria or previously FDA-approved drugs as antibacterial agents for Gram-negative bacteria in the presence of an antibacterial peptide or a membrane-active peptide.

Among many attempts to treat Gram-negative bacteria, antibacterial peptides may be suggested as a substitute for existing antibacterial agents. These antibacterial peptides are products of the immune system and there are various types thereof. The antibacterial peptides are capable of killing bacteria by destroying their membrane. However, due to the membrane-destroying ability, these antibacterial peptides exhibit not only the intended bacteria-killing effect but also toxicity to host cells. Thus, in order to decrease toxicity to host cells, it is necessary to remove the membrane-destroying ability therefrom.

The membrane-active peptide KL-L9P (also referred to as "L9P"), which consists of a total of 14 amino acids of "KLLKLLKKPLKLLK", has been previously known (Korean Patent No. 1811437). Since membrane-destroying ability is proportional to an alpha-helical content, it was expected that an amphipathic peptide having a kinked proline structure would have decreased toxicity to host cells. In fact, it was found that L9P developed in the above-mentioned Korean Patent is a membrane-reorganizing peptide having weak efficacy against *E. coli* while having low toxicity to host cells. That is, presence of the peptide makes it possible to sensitize conventional drugs, that is, hydrophobic antibacterial agents for Gram-positive bacteria or non-antibacterial drugs, which did not exhibit an effective therapeutic effect on Gram-negative bacteria because they stayed in a LPS layer of Gram-negative bacteria and thus were unable to penetrate the membrane, thereby killing Gram-negative bacteria.

However, in order to effectively kill Gram-negative bacteria using the peptide, the peptide needs to be at a high concentration of 4 ug/ml (2.3 uM) or higher. For actual administration to a human body, the peptide needs to be at a large dose of 600 mg. This dose is 5- to 10-fold higher than a usual dose of a drug administered to a human body. Therefore, in order to develop these peptides into actual drugs, there is a need to decrease their dose.

In addition, the peptide needs to be at a relatively large amount of 20 ug/mL in order to have bacteria-reorganizing ability, and there are limited types of hydrophobic antibacterial agents that are sensitized using this peptide. In addition, among Gram-negative bacteria, the peptide tends to easily reorganize the membrane of *E. Coli* or *A. baumannii* while not easily reorganizing the membrane of *K. Pneumoniae* or *P. aeruginosa*.

Under this technical background, the present inventors have developed a peptide or peptide analog, which is capable of exhibiting an enhanced sensitizing effect and at the same time decreased toxicity to host cells, and have identified a use thereof, thereby completing the present disclosure.

[Prior Art Literature]

[Patent Literature]

Korean Patent No. 1811437

**Disclosure of Invention**

**Technical Problem**

The present disclosure intends to provide a peptide or peptide analog having a structure in which an amphipathic, alpha-helical peptide composed of hydrophobic amino acids and hydrophilic amino acids is kinked, with a fatty acid bound to the N-terminus thereof.

In addition, the present disclosure intends to use the peptide or peptide analog for treatment of diseases, in particular, associated with infections caused by Gram-negative bacteria.

**Solution to Problem**

The present disclosure provides a peptide or peptide analog represented by Formula 1:

<Formula 1>  $X_1X_2X_3X_4X_5X_6X_7X_8X_9X_{10}X_{11}X_{12}X_{13}X_{14}X_{15}$

In the formula,

$X_1$, $X_2$, $X_5$, $X_8$, $X_9$, $X_{12}$, and $X_{15}$ are each independently a hydrophilic amino acid or non-proteinogenic amino acid, provided that at least one of them may be Ala or Ser,

$X_3$, $X_4$, $X_6$, $X_7$, $X_{11}$, $X_{13}$, and $X_{14}$ are each independently a hydrophobic amino acid or a mixture thereof,

$X_{10}$ is Pro,

a $C_6$ to $C_{16}$ fatty acid may be bound to any one position of $X_1$ to $X_{15}$, and

$X_1$ is N-terminus and $X_{15}$ is C-terminus.

In addition, the present disclosure provides a pharmaceutical composition for treatment of bacterial infections or an antibacterial pharmaceutical composition, comprising the peptide or peptide analog and a drug.

In addition, the present disclosure provides a conjugate comprising the peptide or peptide analog with a drug linked thereto.

**Advantageous Effects of Invention**

The novel peptide of the present disclosure is capable of specifically acting only on the outer membrane of Gram-negative bacteria to loosen the outer membrane, and thus exhibits an excellent sensitizing effect on existing antibacterial agents. Existing antibacterial agents did not pass through the outer membrane of Gram-negative bacteria due to rigidity of the outer membrane. Thus, these antibacterial agents did not exhibit antibacterial activity against Gram-negative bacteria. However, in a case of being administered in combination with the peptide of the present disclosure, the peptide enables the outer membrane of Gram-negative bacteria to be loosened, which allows existing antibacterial agents to pass through the outer membrane and exhibit antibacterial activity against Gram-negative bacteria. In particular, the peptide of the present disclosure exhibits an excellent sensitizing effect even at a low concentration as compared with known sensitive antibacterial peptides (AMPs), which is advantageous in that the peptide is able to have maximized sensitizing ability even at a low concentration and have decreased adverse effects while delaying appearance of resistant bacteria.

In terms of toxicity, the peptide of the present disclosure exhibits membrane activity specific to Gram-negative bacteria and has a characteristic of binding to a LPS layer on the surface of the outer membrane of Gram-negative bacteria, so that the peptide only stays in the outer membrane and does not exhibit an effect of degrading the outer or inner membrane of Gram-negative bacteria.

In addition, the peptide of the present disclosure makes it possible to accurately screen whether a candidate agent passes into the outer membrane of Gram-negative bacteria and acts as an antibacterial agent. Thus, it is also possible to search for a novel antibacterial agent using this peptide.

**Brief Description of Drawings**

FIG. 1 illustrates amino acid sequences of peptides and their effect of sensitizing antibiotics (erythromycin and novobiocin) against *A. baumannii* ATCC 17978.

FIG. 2 illustrates amino acid sequences of peptides and their effect of sensitizing fusidic acid against A. *baumannii* ATCC 17978.

FIG. 3 illustrates amino acid sequences of peptides and their effect of sensitizing three antibiotics (novobiocin, rifampicin, and fusidic acid) against *A. baumannii* ATCC 17978.

FIG. 4 illustrates amino acid sequences of peptides and their effect of sensitizing three antibiotics (novobiocin, rifampicin, and fusidic acid) against *A. baumannii* ATCC 17978.

FIG. 5 illustrates graphs, showing results obtained by comparing sensitizing effects between L9P (CMP1107) or SPR741 and the peptide of the present disclosure on various types of repositioning antibacterial agents against *E. coli* ATCC 25922.

FIG. 6 illustrates experimental results of $MIC_{50}$ and $MIC_{90}$ observed in a case where rifampicin, colistin, clarithromycin, tedizolid, or linezolid is used alone and in combination with CMP1401 in *A. baumannii* clinical strain.

FIG. 7 illustrates amino acid sequences of peptides and their effect of sensitizing rifampicin or linezolid against *E. coli.* ATCC 25922.

FIG. 8 illustrates amino acid sequences of peptides and their effect of sensitizing rifampicin against four Gram-negative bacteria (A.b. ATCC 17978, K.p. ATCC 700603, E.c. ATCC 25922, and P.a. ATCC 27853).

FIG. 9 illustrates graphs, showing results obtained by comparing sensitizing effects of L9P(CMP1107), CMP1401, and CMP1709 on rifampicin against the four Gram-negative strains E.c. ATCC 25922, A.b. ATCC 17978, K.p. ATCC 700603, and P.a. ATCC 27853, and Carbapenem-resistant Enterobacteriaceae (CRE; that is, Ec NDM-1, Kp NDM-1, and K.p. KPC). The indicated number means a decreased MIC value of rifampicin at a peptide concentration of 4 ug/ml (see table below).

| FIG. | Decreased MIC value (ug/mL) of rifampicin at peptide concentration of 4 ug/ml | |
|---|---|---|
| | CMP1401 | CMP1709 |
| FIG. 9A | 0.0020 | 0.0020 |
| FIG. 9B | 0.031 | 0.078 |
| FIG. 9C | 0.0078 | 0.0078 |
| FIG. 9D | 0.25 | 0.25 |
| FIG. 9E | 0.0040 | 0.0040 |
| FIG. 9F | 0.0078 | 0.0078 |
| FIG. 9G | 0.0078 | 0.0078 |

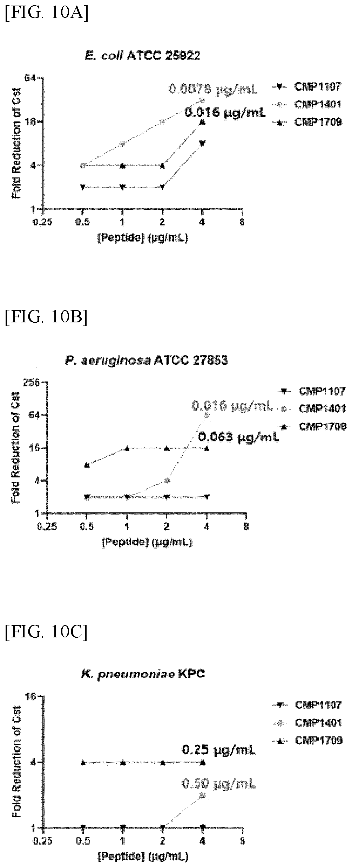

FIG. 10 illustrates graphs, showing results obtained by comparing sensitizing effects of L9P(CMP1107), CMP1401, and CMP1709 on colistin against the three Gram-negative strains E.c. ATCC 25922, P.a. ATCC 27853, and K.p. KPC (CRE). The indicated number means a decreased MIC value of colistin at a peptide concentration of 4 ug/ml (see table below).

| FIG. | Decreased MIC value (ug/mL) of colisitin at peptide concentration of 4 ug/ml | |
|---|---|---|
| | CMP1401 | CMP1709 |
| FIG. 10A | 0.0078 | 0.016 |
| FIG. 10B | 0.016 | 0.063 |
| FIG. 10C | 0.25 | 0.50 |

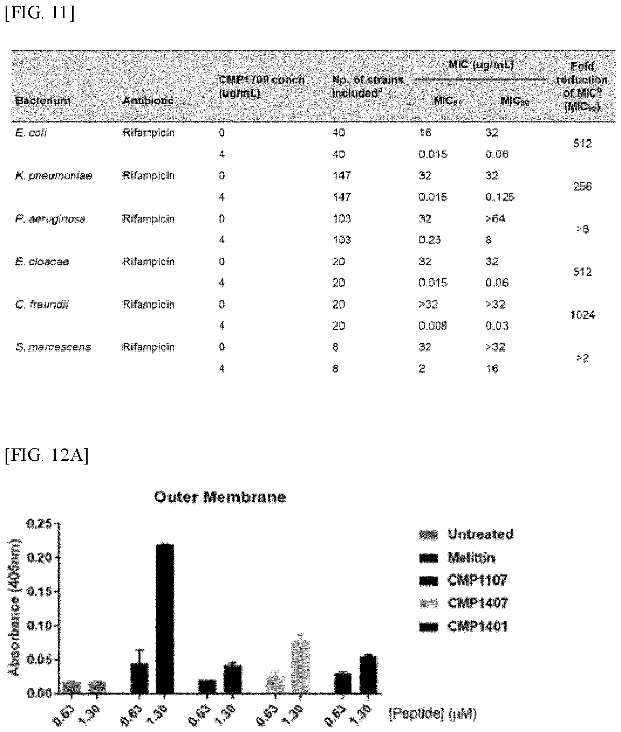

FIG. 11 illustrates experimental results of $MIC_{50}$ and $MIC_{90}$ observed in a case where rifampicin is used alone and in combination with CMP1709 in *E. coli, K. pneumoniae, P. aeruginosa, E. cloacae, C. freundii,* or *S. marcescens* clinical strain.

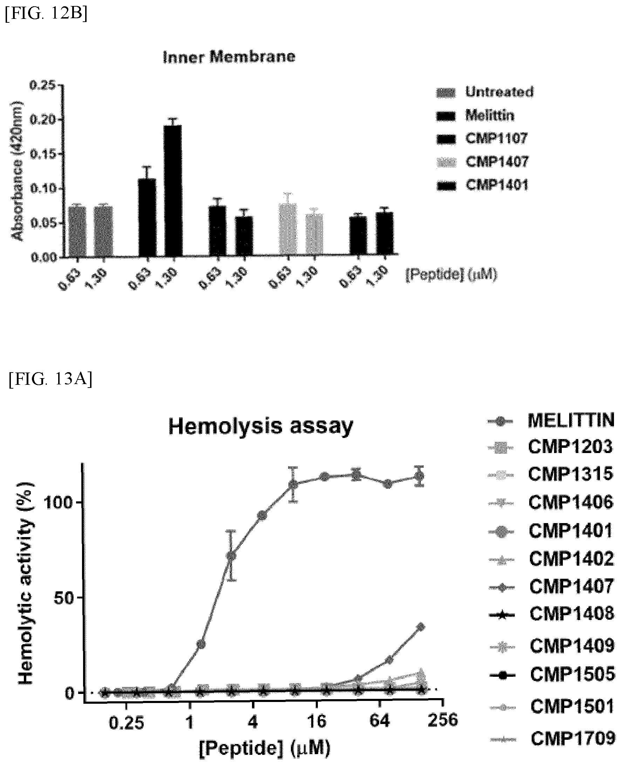

FIG. 12 illustrates results of enzyme assay for L9P (CMP1107), CMP1401, and CMP1407. The strain used for the outer membrane was E. *coli* NDM-1 in which CENTA was used as an enzyme substrate, and the strain used for the inner membrane was *E. coli* ATCC 25922 in which ONPG was used as an enzyme substrate.

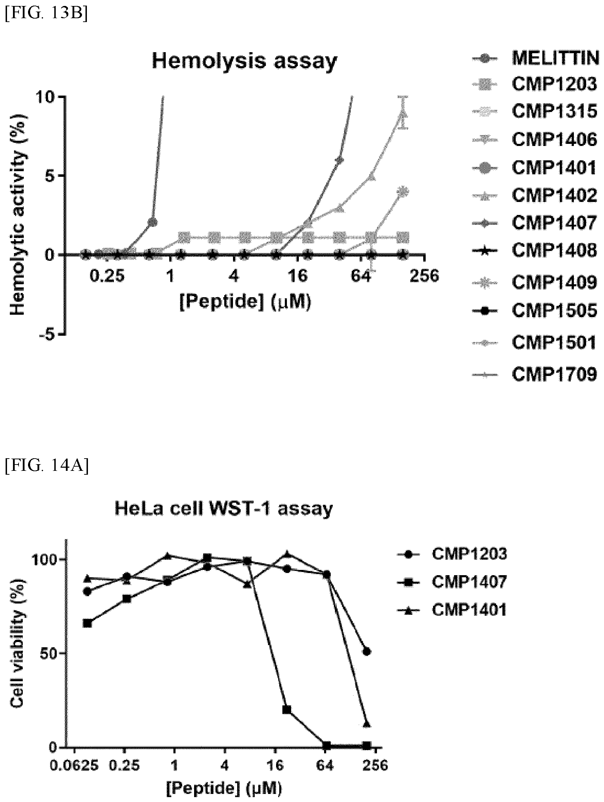

FIG. 13 illustrates results of hemolysis assay for hRBCs of the peptides of the present disclosure. FIG. 13A: All other graphs except for MELITTIN and CMP1407 exhibit very low hemolytic activity, and thus are almost overlapped. FIG. 13B: In the graphs of FIG. 13A, the hemolytic activity is enlarged in a section ranging from 0 to 10%. All other graphs except for MELITTIN, CMP1407, CMP1406, CMP1409, and CMP1203 are almost overlapped close to zero.

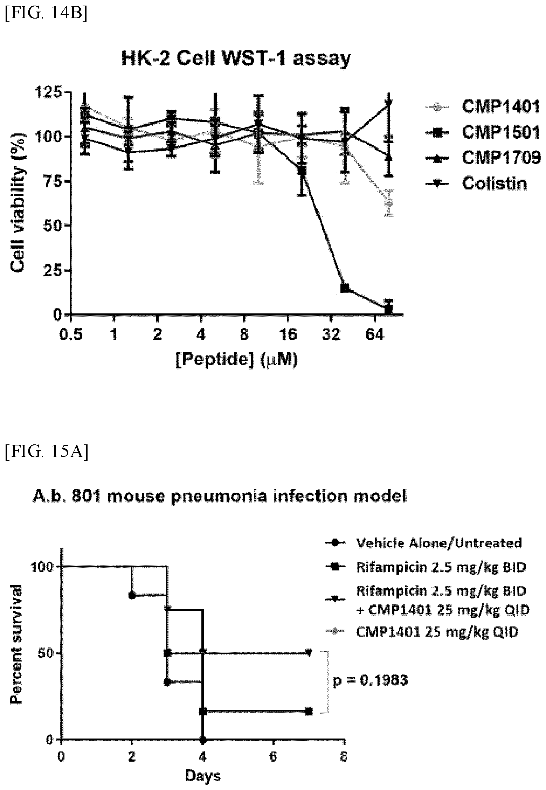

FIG. 14 illustrates results of WST-1 assay for HeLa cells or HK-2 cells of the peptides of the present disclosure. FIG. 14A: Respective graphs show CMP1203, CMP1401, and CMP1407 from top to bottom based on data of 256 uM. FIG. 14B: Respective graphs show colistin, CMP1709, CMP1401, and CMP1501 from top to bottom based on data of 64 uM.

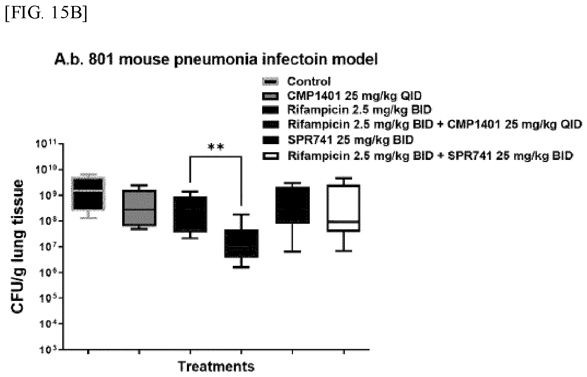

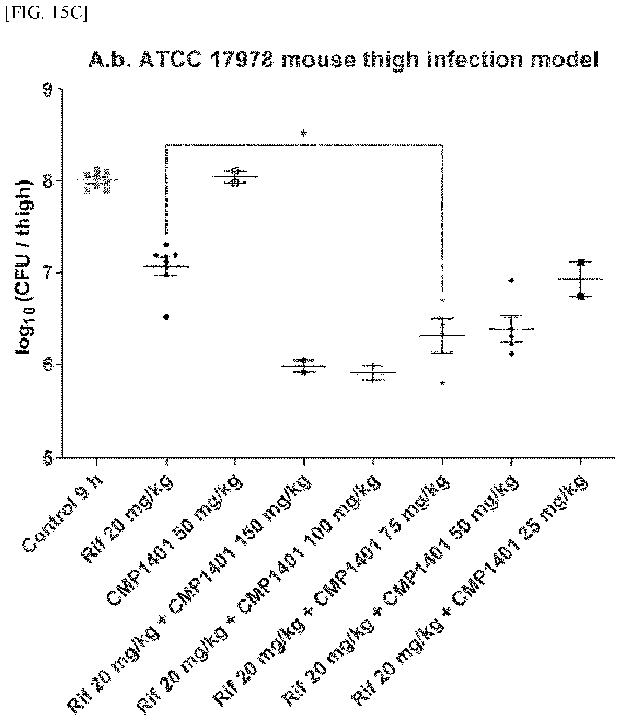

FIG. 15 illustrates *in vivo* data for CMP1401 in a mouse animal model. FIGS. 15A and 15B illustrate effects of co-administration of rifampicin and CMP1401 peptide in an immunosuppressed mouse A. *baumannii* 801 pneumonia infection survival model. FIG. 15C illustrates effects of co-administration of rifampicin and CMP1401 peptide in an immunosuppressed mouse *A. baumannii* ATCC 17978 thigh infection model.

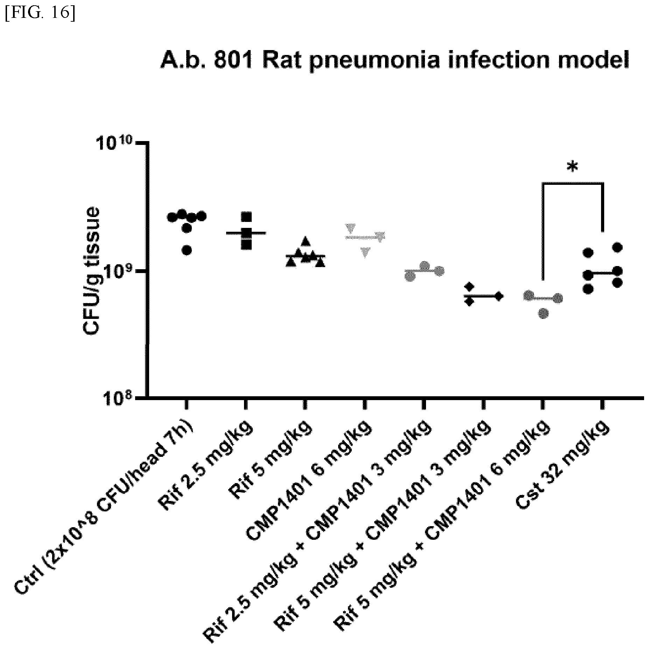

FIG. 16 illustrates effects of co-administration of rifampicin and CMP1401 peptide in an immunosuppressed rat *A. baumannii* 801 pneumonia infection model.

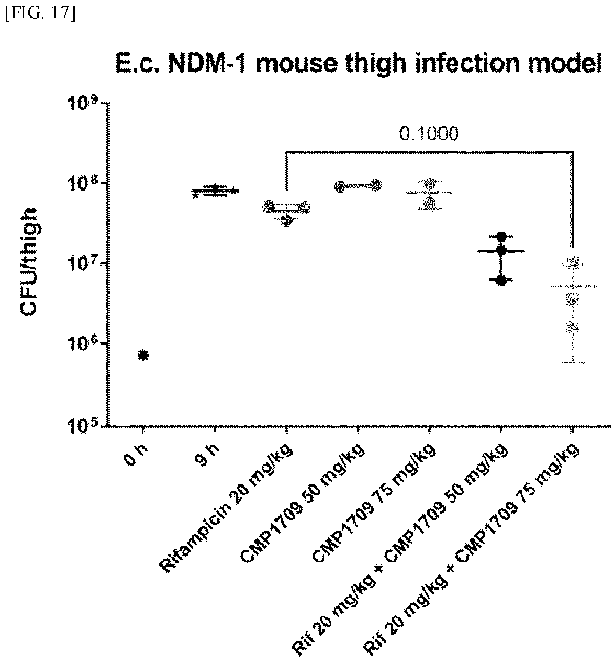

FIG. 17 illustrates effects of co-administration of rifampicin and CMP1709 peptide in an immunosuppressed mouse *E. coli NDM-1* thigh infection model.

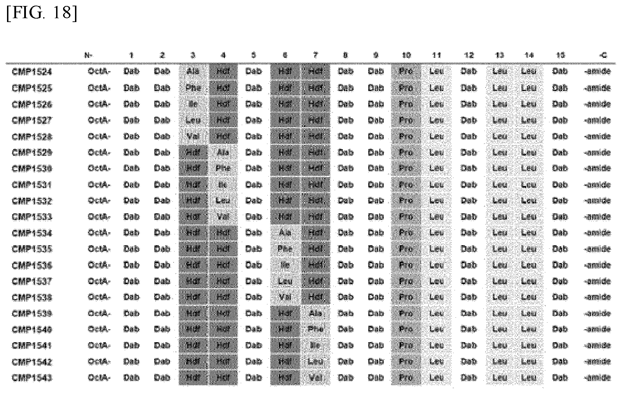

FIG. 18 illustrates fifth-generation sensitizing peptide sequences into which Hdf is inserted. Hdf means that the five hydrophobic amino acids Ala, Ile, Leu, Phe, and Val are inserted into a corresponding site at the same ratio.

## Best Mode for Carrying out Invention

### Peptide or peptide analog

The present disclosure provides a peptide or peptide analog represented by Formula 1:

$$\text{<Formula 1>} \qquad X_1X_2X_3X_4X_SX_6X_7X_8X_9X_{10}X_{11}X_{12}X_{13}X_{14}X_{15}$$

In the above formula,

$X_1$, $X_2$, $X_5$, $X_8$, $X_9$, $X_{12}$, and $X_{15}$ are each independently a hydrophilic amino acid or non-proteinogenic amino acid, provided that at least one of them may be Ala or Ser,

$X_3$, $X_4$, $X_6$, $X_7$, $X_{11}$, $X_{13}$, and $X_{14}$ are each independently a hydrophobic amino acid or a mixture thereof,

$X_{10}$ is Pro,

a $C_6$ to $C_{16}$ fatty acid may be bound to any one position of $X_1$ to $X_{15}$, and

$X_1$ is N-terminus and $X_{15}$ is C-terminus.

Specifically, the $C_6$ to $C_{16}$ fatty acid may be, for example, hexanoic acid, heptanoic acid, octanoic acid, decanoic acid, or lauric acid, but is not limited thereto.

The peptide or peptide analog of the present disclosure has a structure in which an amphipathic, alpha-helical peptide composed of hydrophobic amino acids and hydrophilic amino acids is kinked, with a fatty acid bound to the N-terminus thereof.

In the present specification, the term "kinked" may be used in the same sense as "folded", "bent", "curved", or broken", and the "kinked" structure may be a structure formed by substitution of some hydrophobic amino acids in the amphipathic, alpha-helical peptide composed of hydrophobic amino acids and hydrophilic amino acids, and this structure may be in a form where an alpha helix is bent around the amino acid-substituted portion in the amphipathic, alpha-helical peptide.

In a specific embodiment, $X_1$, $X_2$, $X_5$, $X_8$, $X_9$, $X_{12}$, and $X_{15}$ may each independently be a hydrophilic amino acid, a non-proteinogenic amino acid, Ala, or Ser.

In a specific embodiment, $X_1$, $X_2$, $X_5$, $X_8$, $X_9$, $X_{12}$, and $X_{15}$ may each independently be a hydrophilic amino acid selected from Lys, Arg, His, and derivatives thereof, or 2,3-diaminopropionic acid (Dap), 2,4-diaminobutanoic acid (Dab), or ornithine (Om), provided that at least one of them may be Ala or Ser; and

$X_3$, $X_4$, $X_6$, $X_7$, $X_{11}$, $X_{13}$, and $X_{14}$ may each independently be a hydrophobic amino acid selected from Leu, Ala, Ile, Phe, Val, Trp, or Tyr, or Hdf, wherein Hdf may be a mixture of amino acids comprising Leu, Ala, Val, Ile, and Phe in equal amounts.

In another embodiment, $X_1$, $X_2$, $X_5$, $X_8$, $X_9$, $X_{12}$, and $X_{15}$ may each independently be a hydrophilic amino acid selected from Lys, Arg, His, and derivatives thereof, or 2,3-diaminopropionic acid (Dap), 2,4-diaminobutanoic acid

**EP 4 134 092 A1**

(Dab), ornithine (Om), Ala, or Ser; and

$X_3$, $X_4$, $X_6$, $X_7$, $X_{11}$, $X_{13}$, and $X_{14}$ may each independently be a hydrophobic amino acid selected from Leu, Ala, Ile, Phe, Val, Trp, or Tyr, or Hdf, wherein Hdf may be a mixture of amino acids comprising Leu, Ala, Val, Ile, and Phe in equal amounts.

In yet another embodiment, $X_1$, $X_2$, $X_5$, $X_8$, $X_9$, $X_{12}$, and $X_{15}$ may each independently be Lys,Ala, Ser, or 2,4-diaminobutanoic acid (Dab).

In still yet another embodiment, $X_3$, $X_4$, $X_6$, and $X_7$ may each independently be Leu, Ala, Val, Ile, Phe, or Hdf.

In still yet another embodiment, $X_{11}$, $X_{13}$, and $X_{14}$ may each independently be Leu or Ala.

In still yet another embodiment, a $C_6$ to $C_{12}$ fatty acid may be bound to a position of $X_1$.

Korean Patent No. 1811437 discloses a sensitizing peptide that exhibits an enhanced sensitizing effect on existing drugs against Gram-negative bacteria and the like. However, since the peptide disclosed therein alone does not achieve sufficient sensitizing effect and toxicity decreasing effect, the present inventors intended to provide a novel peptide or peptide analog, which is most optimized for a combination therapy with an antibacterial agent, as a strategy to cause the peptide to have decreased toxicity and at the same time enhanced sensitizing effect.

As used herein, the term "peptide" is an amino acid polymer, which may comprise, as constituent elements, not only natural amino acids but also non-proteinaceous amino acids.

The present disclosure also includes a "peptide analog". The peptide analog may include analogs obtained by substitution with one or more other functional groups on amino acid side chains or an alpha-amino acid backbone. Examples of side chain- or backbone-modified peptide analogs include, but are not limited to, hydroxyproline in which a pyrrolidine ring is substituted with a hydroxyl group, or N-methylglycine "peptoid". Types of peptide analogs are known in the art.

The "peptide" or the "peptide analog" is also collectively referred to herein as a "peptide".

In a specific embodiment, the peptide of the present disclosure may be represented by Formula 1 wherein $X_1$, $X_2$, $X_5$, $X_8$, $X_9$, $X_{12}$, and $X_{15}$ are each independently Lys, and $X_3$, $X_4$, $X_6$, $X_7$, $X_{11}$, $X_{13}$, and $X_{14}$ are each independently Leu (Formula 1b). A representative peptide included in the peptide of this general formula (Formula 1b) may consist of the amino acid sequence of SEQ ID NO: 1. As compared with the known peptide KL-L9P, this peptide has an enhanced level of interaction with a phospholipid layer of a wide spectrum of Gram-negative bacteria including E. *coli.* Furthermore, due to an increased number of positive charges in the peptide, a lower concentration of the peptide acts more favorably on interaction with LPS distributed in the outer membrane of Gram-negative strains. By selecting a total length of 15 amino acids, it is possible to decrease a risk of cell membrane degradation that is caused due to an increase in length in a case where a too long membrane-active peptide is used.

In another specific embodiment, the peptide of the present disclosure may be represented by Formula 1 wherein $X_1$, $X_2$, $X_5$, $X_8$, $X_9$, $X_{12}$, and $X_{15}$ are each independently Lys or Ala, and $X_3$, $X_4$, $X_6$, $X_7$, $X_{11}$, $X_{13}$, and $X_{14}$ are each independently Leu, Ala or Val, provided that at least one of $X_1$ to $X_{15}$ except for $X_{10}$ is Ala or Val (Formula 1c). A representative peptide included in the peptide of this general formula (Formula 1c) may consist of the amino acid sequence of any one of SEQ ID NOs: 2 to 17 and61.

In a case where an amphipathic peptide comprises Ala or Val, in particular, Ala, as in the peptide of Formula 1c, the amphipathic peptide has decreased overall hydrophobicity and decreased size of hydrophobic side chain residue, which may affect a way how a hydrophobic surface of a membrane-active peptide binds to phospholipid of the outer membrane of Gram-negative bacteria, and thus may cause the peptide exhibit stronger membrane activity even at a lower concentration.

In yet another specific embodiment, the peptide of the present disclosure may be represented by Formula 1 wherein $X_1$, $X_2$, $X_5$, $X_8$, $X_9$, $X_{12}$, and $X_{15}$ are each independently Lys,$X_3$, $X_7$, and $X_{11}$ are each independently Leu, Ala, or Val, $X_4$, $X_6$, $X_{13}$, and $X_{14}$ are each independently Leu, and a $C_6$ to $C_{12}$ fatty acid is bound to a position of $X_1$ (Formula 1d). A representative peptide included in the peptide of this general formula (Formula 1d) may consist of the amino acid sequence of any one of SEQ ID NOs: 18 to 26 and 62 to 63.

In a case where a peptide has a lipidated chain as in the peptide of Formula 1d, the peptide may have increased ability of binding to a protein present in plasma or blood, thereby achieving increased PK and at the same time enhanced sensitizing ability. In the peptide, lipidation may be made at several positions. Preferably, lipidationmay be made at the N-terminus. A length of the fatty acid may vary from $C_6$ to $C_{16}$, with $C_6$ to $C_{12}$ being preferred. In particular, in a case where a length of $C_6$ to $C_8$ is used, the greatest change in sensitization activity is observed, and lipidation-induced peptide toxicity as measured by hemolytic activity is hardly increased.

In still yet another specific embodiment, the peptide of the present disclosure may be represented by Formula 1 wherein $X_1$, $X_2$, $X_5$, $X_8$, $X_9$, $X_{12}$, and $X_{15}$ are each independently Dab, $X_3$, $X_4$, $X_6$, and $X_7$ are each independently Leu, Ala, Ile, Phe, Val, or Hdf, wherein at least two of $X_3$, $X_4$, $X_6$, and $X_7$ are each independently Ala, Ile, Phe, Val, or Hdf, $X_{11}$, $X_{13}$, and $X_{14}$ are each independently Leu, and a $C_8$ or $C_{12}$ fatty acid is bonded to a position of $X_1$ (Formula 1e). A

representative peptide included in the peptide of this general formula (Formula 1e) may consist of the amino acid sequence of any one of SEQ ID NOs: 27 to 48 and 64 to 66.

Formula 1e has diversity in hydrophilic and hydrophobic surfaces. First, in a case where the hydrophilic surface comprises ornithine (Om), diaminobutyric acid (Dab), diaminopropionic acid (Dap), or the like having a non-natural primary amine residue, the peptide may have a preserved or enhanced membrane-reorganizing effect. In a case where Dab is included therein, the peptide may have an enhanced sensitizing effect, in particular against *P. aeruginosa.* In particular, in a case where Dab is included therein, the peptide may have decreased hemolytic activity that may be caused by lipidation, thereby exhibiting decreased toxicity.

In a case where the five amino acids Leu, Ala, Ile, Val, and Phe are mixed at a time and used as a mixture (Hdf) in the hydrophobic surface, the peptide exhibits excellent membrane-reorganizing ability.

In still yet another specific embodiment, the peptide of the present disclosure may be represented by Formula 1 wherein $X_1$, $X_2$, $X_5$, $X_8$, $X_9$, $X_{12}$, and $X_{15}$ are each independently Dab, $X_3$, $X_4$, $X_6$, and $X_7$ are each independently Leu, Ala, Phe, or Val, wherein at least three of $X_3$, $X_4$, $X_6$, and $X_7$ are each independently Ala, Phe, or Val, $X_{11}$, $X_{13}$, and $X_{14}$ are each independently Leu, and a $C_8$ fatty acid is bound to a position of $X_1$ (Formula 1f). A representative peptide included in the peptide of this general formula (Formula 1f) may consist of the amino acid sequence of any one of SEQ ID NOs: 49 to 52.

In still yet another specific embodiment, the peptide of the present disclosure may be represented by Formula 1 wherein $X_1$, $X_2$, $X_5$, $X_8$, $X_9$, $X_{12}$, and $X_{15}$ are each independently Dab or Ser, wherein at least one of $X_1$, $X_2$, $X_5$, $X_8$, $X_9$, $X_{12}$, and $X_{15}$ is each independently Ser, $X_3$ and $X_7$ are each independently Ala, $X_4$ and $X_6$ are each independently Leu, Phe or Val, $X_{11}$, $X_{13}$, and $X_{14}$ are each independently Leu, and a $C_8$ fatty acid is bound to a position of $X_1$ (Formula 1g). A representative peptide included in the peptide of this general formula (Formula 1g) may consist of the amino acid sequence of any one of SEQ ID NOs: 53 to 60 and 67.

In still yet another embodiment, the peptide of the present disclosure may be represented by Formula 1 wherein $X_1$, $X_2$, $X_5$, $X_8$, $X_9$, $X_{12}$, and $X_{15}$ are each independently Lys, Ala, Ser, or Dab, $X_3$, $X_4$, $X_6$, and $X_7$ are each independently Leu, Ala, Val, Ile, Phe, or Hdf, $X_{10}$ is Pro, $X_{11}$, $X_{13}$, and $X_{14}$ are each independently Leu or Ala, and a $C_6$ to $C_{12}$ fatty acid is bound to a position of $X_1$.

In a specific embodiment, the peptide may be a peptide or peptide analog, characterized by comprising the amino acid sequence of any one of SEQ ID NOs: 1 to 67.

The peptide or peptide analog of the present disclosure may exhibit any of the following characteristics.

i) It has activity against Gram-negative bacteria without having hemolytic activity against host cells or activity against Gram-positive bacteria;

ii) it has a characteristic capable of binding to an LPS layer on the surface of the outer membrane of Gram-negative bacteria;

iii) it has a characteristic capable of binding to an LPS layer on the surface of the outer membrane of Gram-negative bacteria while entering the outer membrane and staying only therein;

iv) it has a characteristic of penetrating the outer membrane of Gram-negative bacteria and staying only in the outer membrane while having no ability to degrade the outer or inner membrane of Gram-negative bacteria;

v) it has a structure in which alpha-helix is kinked by Pro, which makes it possible to have minimized toxicity to host cells while having increased recognition of the outer membrane of Gram-negative bacteria;

vi) it is positively charged and has hydrophobicity at the same time, which makes it possible to have maximized recognition of negative charges and hydrophobicity on the outer membrane (LPS layer) of Gram-negative bacteria; and

vii) it has increased half-life in a body through lipidation at the N-terminus, in which the lipidated portion and hydrophobic residues are able to be harmoniously combined.

The peptide according to the present disclosure may be prepared according to a conventionally known method, for example, a solid-phase peptide synthesis method. This preparing method further forms an embodiment of the present disclosure.

**Antibacterial application**

The peptide or peptide analog of the present disclosure may be used for antibacterial application such as treatment of bacterial infections.

Accordingly, in an embodiment, the present disclosure provides a method of preventing, ameliorating, or treating a bacterial infection, comprising a step of administering to a subject an effective amount of the peptide or peptide analog. As used herein, the phrase "preventing, ameliorating, or treating a bacterial infection" may be used interchangeably with "antibacterial".

In another embodiment, the present disclosure provides a use of the peptide or peptide analog for prevention, amelioration, or treatment of a bacterial infection.

In yet another embodiment, the present disclosure provides a pharmaceutical composition for preventing, ameliorating, or treating a bacterial infection, comprising the peptide or peptide analog.

As used herein, the term "subject" is meant to include both human and non-human animals. The non-human animal includes all vertebrates, for example, mammals and non-mammals, including non-human primates, sheep, dogs, cats, cattle, horses, chickens, amphibians, and reptiles. For example, mammals such as non-human primates, sheep, dogs, cats, cattle, and horses are preferred. A preferred subject is a human in need of prevention or treatment of cancer.

The bacterial infection may be, preferably, a Gram-negative bacterial infection. Accordingly, the peptide or peptide analog of the present disclosure may be used to exhibit antibacterial activity against Gram-negative bacteria. Here, the Gram-negative bacteria means pathogenic microorganisms or resistant bacteria, preferably Gram-negative bacterial pathogenic microorganisms or Gram-negative resistant bacteria. Examples of Gram-negative bacteria include, but are not limited to, E. *coli, Acinetobacter baumannii, Klebsiella pneumoniae, Pseudomonas aeruginosa, Enterobacter cloacae, Citrobacter freundii,* and *Serratia marcescens.*

In a preferred embodiment, the peptide of the present disclosure is used in combination with another drug. Accordingly, the present disclosure provides a pharmaceutical composition for treatment of bacterial infections or an antibacterial pharmaceutical composition, comprising the peptide or peptide analog, and a drug. Here, the peptide or peptide analog, and the drug may be administered simultaneously as one formulation, or administered simultaneously or sequentially as separate formulations. Specifically, in a case where the peptide or peptide analog of the present disclosure is used in combination with another drug, they may be administered separately or may be applied in the form of a combination product in which a plural ity of active ingredients are present in one pharmaceutical formulation. In a case where they are administered as separate formulations, the two formulations may be administered sequentially or simultaneously. For simultaneous administration, these formulations are given together to a patient. For sequential administration, these formulations may be given at a time interval that is not long. For example, these formulations may be administered to a patient within a period of 12 hours or less, or 6 hours or less.

In another embodiment, the present disclosure provides a method for preventing, ameliorating, or treating a bacterial infection, comprising a step of administering an effective amount of the peptide or peptide analog in combination with a drug. The above embodiment includes simultaneous administration using one composition that comprises the peptide and the drug together, as well as simultaneous or sequential administration using separate compositions, each of which comprises either of the peptide and the drug, to a patient in need thereof.

In yet another embodiment, the present disclosure provides a use of the peptide or peptide analog in combination with a drug, for prevention, amelioration, or treatment of a bacterial infection.

The drug may be a drug that is conventionally used as an antibacterial agent for Gram-positive bacteria, a drug that has been approved by US FDA or the like for other uses but not approved as an antibacterial agent, or a drug used for Gram-negative bacteria. Examples of the antibacterial agent for Gram-positive bacteria may include, but are not limited to, rifampicin, rifabutin, rifaximin, rifapentine, tedizolid, linezolid, clarithromycin, telithromycin, retapamulin, mupirocin, erythromycin, fusidic acid, or novobiocin. The drug used for Gram-negative bacteria may include, but is not limited to, colistin, aztreonam, azithromycin, ceftazidime, ciprofloxacin, chloramphenicol, gentamycin, trimethoprim, nalidixic acid, or levofloxacin.

More specifically, examples of the drug, which may be used in combination with the peptide of the present disclosure, include, but are not limited to, erythromycin, novobiocin, fusidic acid, rifampicin, rifaximin, chloroxine, gatifloxacin, lomefloxacin, rifabutin, rifapentine, daptomycin, nisin, tigecycline, aztreonam, ceftazidime, nitrofurantoin, chloramphenicol, fidaxomicin, retapamulin, cefepime, mecillinam, meropenem, vancomycin, clarithromycin, fosfomycin, ramoplanin, ciprofloxacin, gentamycin, tobramycin, linezolid, telithromycin, levofloxacin, trimethoprim, clindamycin, nalidixic acid, azithromycin, mupirocin, daptomycin, linezolid, nitrofurantoin, fidaxomicin, aztreonam, retapamulin, tobramycin, tedizolid, albamycin, auranofin, capitrol, triclosan, butoconazole, miconazole, clioqu inol, lapatinib, sorafenib, bleomycin, quinestrol, aztreonam, or colistin.

As used herein, the term "prevention" refers to any action that entails administration of the composition to suppress or delay onset of infectious diseases caused by pathogenic microorganisms or resistant bacteria, and the term "treatment" refers to any action that entails administration of the composition to ameliorate or beneficially alter symptoms of infectious diseases caused by pathogenic microorganisms or resistant bacteria.

The composition may further comprise a pharmaceutically acceptable carrier. As used herein, the term "pharmaceutically acceptable carrier" refers to a pharmaceutically acceptable material, composition, or vehicle, such as a liquid or solid filler, a diluent, an excipient, a solvent, or a capsulation material, which is involved in conveyance or transportation of any target composition or ingredient from one organ or part of the body to another organ or part of the body. For administration, the composition of the present disclosure may further comprise a pharmaceutically acceptable carrier, excipient, or diluent in addition to the above-mentioned active ingredient. Examples of the carrier, excipient, and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyhydroxybenzoate, propylhydroxybenzoate, talc, magnesium stearate, and mineral oil.

In addition, the composition of the present disclosure may be formulated in the form of oral preparations such as powders, granules, tablets, capsules, suspensions, emulsions, syrups, and aerosols, preparations for external use, suppositories, and sterile injectable solutions, respectively, according to conventional methods, and used. Specifically, in a case of being formulated into preparations, the preparation may be prepared using a commonly used diluent or excipient such as filler, extender, binder, wetting agent, disintegrant, or surfactant. Solid preparations for oral administration include, but are not limited to, tablets, pills, powders, granules, capsules, and the like. Such solid preparations may be prepared by mixing the composition with at least one excipient, such as starch, calcium carbonate, sucrose, lactose, and gelatin. In addition to simple expedients, lubricants such as magnesium stearate and talc may also be used. Liquid preparations for oral administration include, but are not limited to, suspensions, solutions, emulsions, syrups, and the like, and may be prepared by adding various excipients, such as wetting agents, sweeteners, fragrances, and preservatives, in addition to commonly used simple diluents such as water and liquid paraffin. Formulations for parenteral administration include sterile aqueous solutions, non-aqueous solvents, suspensions, emulsions, lyophilized agents, and suppositories. Non-aqueous solvents and suspensions may be prepared using propylene glycol, polyethylene glycol, vegetable oil such as olive oil, or injectable ester such as ethyloleate. As a base for suppositories, Witepsol, Macrogol, Tween 61, cacao fat, laurin fat, glycerogelatin, and the like may be used.

The composition according to the present disclosure may be administered orally or parenterally (for example, intravenously, subcutaneously, intraperitoneally, or topically) according to a desired method. A dosage of the composition may vary depending on the patient's condition and weight, severity of disease, type of drug, and administration route and period of administration, and may be appropriately selected by a person skilled in the art. The composition may be administered once or several times a day as needed, and may be used alone or in combination with methods using surgery, hormone therapy, drug treatment, and biological response modifiers for prevention or treatment of diseases caused by pathogenic bacteria and resistant bacteria.

As used herein, the term "co-administration" may be used interchangeably with combined administration. A mode of co-administration may include both a mode in which the peptide or peptide analog is administered simultaneously with another compound, and a mode in which the peptide or peptide analog is administered separately from another compound. Here, the peptide or peptide analog according to the present inventionmay be co-administered with one or more selected from the group consisting of a hydrophobic compound having a log P (partition coefficient) value of 0.19 or higher, a compound positively charged under a physiological pH condition, and colistin.

The hydrophobic compound having a log P (partition coefficient) value of 0.19 or higher may be, for example, cloxacillin, linezolid, resveratrol, curcumin, quercetin, simvastatin, lovastatin, mevastatin, catechin, or thymol, but is not limited thereto.

The compound positively charged under a physiological pH condition, for example, pH 7.3 to pH 7.4, may be, for example, erythromycin, rifampicin, colistin, polymyxin B, or nicotine, but is not limited thereto.

A compound negatively charged under a physiological pH condition, for example, pH 7.3 to pH 7.4, may be, for example, ibuprofen, atorvastatin, fluvastatin, pravastatin, carprofen, trans-ferulic acid, or bromfenac, but is not limited thereto.

In an embodiment, a compound that exhibits a synergistic effect in a case of being co-administered with the peptide or peptide analog according to the present disclosure may be, for example, linezolid, erythromycin, ibuprofen, simvastatin, curcumin, or resveratrol, in which the synergistic effect means, for example, that the compound exhibits an antibacterial effect even at a significantly low concentration as compared with a case where effects resulting from a plurality of the peptides or peptide analogs or the compounds to be co-administered are simply additively combined.

In another aspect, the present disclosure relates to a conjugate comprising the peptide or peptide analog and a drug linked to the peptide or peptide analog.

The drug may be a hydrophobic compound having a log P (partition coefficient) value of 0.19 or higher, a compound positively charged under a physiological pH condition, or colistin. A definition of each ingredient is the same as mentioned above.

The peptide or peptide analog and the drug may be linked to each other via, for example, a non-covalent bond or a covalent bond. The non-covalentbondmay be, for example, at least one selected from the group consisting of hydrogen bond, electrostatic interaction, hydrophobic interaction, van der Waals interaction, pi-pi interaction, and cation-pi interaction. The covalent bond may be either a degradable bond or a non-degradable bond. The degradable bond may be a disulfide bond, an acid-degradable bond, an ester bond, an anhydride bond, a biodegradable bond, or an enzyme-degradable bond, but is not limited thereto. The non-degradable bond may be an amide bond or a phosphate bond, but is not limited thereto.

Hereinafter, the present disclosure will be described in more detail by way of examples. These examples are only for illustrating the present disclosure, and it will be apparent to those of ordinary skill in the art that the scope of the present disclosure is not to be construed as being limited by these examples.

**Mode for Carrying out Invention**

**Production examples. Peptide synthesis**

Peptides of SEQ ID NOs: 1 to 67 were synthesized using a solid-phase peptide synthesis method.

Specifically, the synthesis was carried out using a standard fluorenylmethyloxy carbonyl (Fmoc) solid-phase peptide in a peptide microwave synthesizer (CEM). Rink amide MBHA resin (0.59 mmole/g loading, 50 mg, 29.5 $\mu$mol) was used in Discover SPS. The resin was deprotected with 20% piperidine in DMF. Coupling reaction was performed using amino acids of each sequence, PyBOP, and N-diisopropylethylamine (DIPEA). The peptide was isolated from the resin using a cleavage cocktail for 2 hours at room temperature (in which 950 $\mu$L of trifluoroacetic acid, 25 $\mu$L of triisopropylsilane, and 25 $\mu$L of water were used). The isolated peptide was precipitated with n-hexane and diethyl ether (v/v = 1/1) and purified by reverse phase chromatography HPLC. Purification was performed using HPLC (Agilent HPLC 1100 series) with a Zorbax C18 column (3.5 $\mu$m, 4.6×150 mm).

HPLC conditions: buffer A (water with 0.1 % v/v TFA) and buffer B (acetonitrile with 0.1 % v/v TFA), flow rate of 1 mL/min; 0 min, 0% B followed by a linear gradient of 100% B over 60 minutes.

The respective peptides were named as in Table 1.

[Table 1]

| SEQ ID NO | Name | SEQ ID NO | Name | SEQ ID NO | Name | SEQ ID NO | Name |
|---|---|---|---|---|---|---|---|
| 1 | CMP1203 | 21 | CMP1404 | 41 | CMP1536 | 61 | CMP1316 |
| 2 | CMP1301 | 22 | CMP1405 | 42 | CMP1537 | 62 | CMP1410 |
| 3 | CMP1302 | 23 | CMP1406 | 43 | CMP1538 | 63 | CMP1411 |
| 4 | CMP1303 | 24 | CMP1407 | 44 | CMP15391 | 64 | CMP1516. |
| 5 | CMP1304 | 25 | CMP1408 | 45 | CMP1540 | 65 | CMP1517 |
| 6 | CMP1305 | 26 | CMP1409 | 46 | CMP1541 | 66 | CMP1518 |
| 7 | CMP1306 | 27 | CMP1501 | 47 | CMP1542 | 67 | CMP 1708 |
| 8 | CMP1307 | 28 | CMP1505 | 48 | CMP1543 | | |
| 9 | CMP1308 | 29 | CMP1524 | 49 | CMP1601 | | |
| 10 | CMP1309 | 30 | CMP1525 | 50 | CMP1602 | | |
| 11 | CMP131C | 31 | CMP1526 | 51 | CMP1603 | | |
| 12 | CMP1311 | 32 | CMP1527 | 52 | CMP1604 | CMP1604 | |
| 13 | CMP1312 | 33 | CMP1528 | 53 | CMP1701 | | |
| 14 | CMP1313 | 34 | CMP1529 | 54 | CMP1702 | | |
| 15 | CMP1314 | 35 | CMP153C: | 55 | CMP1703 | | |
| 16 | CMP1315 | 36 | CMP1531 | 56 | CMP1704 | CMP1704 | |
| 17 | CMP1317 | 37 | CMP1532 | 57 | CMP1705 | | |
| 18 | CMP1401 | 38 | CMP1533 | 58 | CMP1706 | CMP1706 | |
| 19 | CMP 1402 | 39 | CMP 1534 | 59 | CMP 1707 | | |
| 20 | CMP 1403 | 40 | CMP 1535 | 60 | CMP1709 | | |

Sequences of these peptides, and their calculated and observed values of MALDI TOF mass are shown in Table 2. The mass values of CMP1524 to CMP1543, which correspond to a deconvolution library, were indicated as a range. (Hdf = equal amount of five hydrophobic amino acids A, F, I, L, and V)

[Table 2]

| Peptide | Sequence | [M+H]+ (calcd.) | [M+H]+ (obsd.) |
|---|---|---|---|
| CMP1107 | Ac-KLLKLLKKPLKLLK-NH$_2$ | 1719.23 | 1718.56 |
| CMP 1203 | Ac-KKLLKLLKKPLKLLK-NH$_2$ | 1846.53 | 1846.92 |
| CMP1301 | Ac-AKLLKLLKKPLKLLK-NH$_2$ | 1788.29 | 1788.362 |
| CMP1302 | Ac-KALLKLLKKPLKLLK-NH$_2$ | 1788.29 | 1788.225 |
| CMP1303 | Ac-KKALKLLKKPLKLLK-NH$_2$ | 1803.3 | 1803.682 |
| CMP1304 | Ac-KKLAKLLKKPLKLLK-NH$_2$ | 1803.3 | 1803.352 |
| CMP 1305 | Ac-KKLLALLKKPLKLLK-NH$_2$ | 1788.29 | 1788.499 |
| CMP 1306 | Ac-KKLLKALKKPLKLLK-NH$_2$ | 1803.3 | 1803.379 |
| CMP 1307 | Ac-KKLLKLAKKPLKLLK-NH$_2$ | 1803.3 | 1803.558 |
| CMP1308 | Ac-KKLLKLLAKPLKLLK-NH$_2$ | 1788.29 | 1788.394 |
| CMP1309 | Ac-KKLLKLLKAPLKLLK-NH$_2$ | 1788.29 | 1788.467 |
| CMP1310 | Ac-KKLLKLLKKPAKLLK-NH$_2$ | 1803.3 | 1803.502 |
| CMP1311 | Ac-KKLLKLLKKPLALLK-NH$_2$ | 1788.29 | 1788.275 |
| CMP1312 | Ac-KKLLKLLKKPLKALK-NH$_2$ | 1803.3 | 1803.245 |
| CMP1313 | Ac-KKLLKLLKKPLKLAK-NH$_2$ | 1803.3 | 1803.432 |
| CMP1314 | Ac-KKLLKLLKKPLKLLA-NH$_2$ | 1788.29 | 1789.558 |
| CMP1315 | Ac-KKALKLAKKPLKLLK-NH$_2$ | 1761.25 | 1761.367 |
| CMP1316 | Ac-KKLLKLVKKPLKLLK-NH$_2$ | 1832.31 | 1831.357 |
| CMP1317 | Ac-KKVLKLVKKPLKLLK-NH$_2$ | 1817.31 | 1817.669 |
| CMP1401 | OctA-KKALKLAKKPLKLLK-NH$_2$ | 1846.34 | 1846.435 |
| CMP1402 | HexA-KKLLKLLKKPLKLLK-NH$_2$ | 1901.41 | 1901.15 |
| CMP 1403 | HexA-KKLLKLAKKPLKLLK-NH$_2$ | 1859.36 | 1859.587 |
| CMP1404 | HexA-KKLLKLVKKPLKLLK-NH$_2$ | 1887.39 | 1887.818 |
| CMP 1405 | HexA-KKVLKLVKKPLKLLK-NH$_2$ | 1873.37 | 1873.991 |
| CMP1406 | HexA-KKALKLAKKPLKLLK-NH$_2$ | 1817.31 | 1817.512 |

(continued)

| Peptide | Sequence | [M+H]$^+$ (calcd.) | [M+H]$^+$ (obsd.) |
|---|---|---|---|
| CMP1407 | OctA-KKLLKLLKKPLKLLK-NH$_2$ | 1929.44 | 1929.424 |
| CMP1408 | DecA-KKALKLAKKPLKLLK-NH$_2$ | 1873.37 | 1874.074 |
| CMP1409 | LauA-KKALKLAKKPLKLLK-NH$_2$ | 1901.41 | 1902.53 |
| CMP1410 | MyrA-KKALKLAKKPLKLLK-NH$_2$ | 1929.44 | 1929.091 |
| CMP1411 | PalA-KKALKLAKKPLKLLK-NH$_2$ | 1957.47 | 1957.1 |
| CMP1501 | OctA-DabDabALDabLADabDabPLDabLLDab-NH$_2$ | 1649.12 | 1650.44 |
| CMP1516 | HexA-DabDabALDabLADabDabPLDabLLDab-NH$_2$ | 1620.09 | 1622.11 |
| CMP1517 | DecA-DabDabALDabLADabDabPLDabLLDab-NH$_2$ | 1676.16 | 1678.212 |
| CMP1518 | LauA-DabDabALDabLADabDabPLDabLLDab-NH$_2$ | 1704.19 | 1706.25 |
| CMP1524 | OctA-DabDabA(Hdf)Dab(Hdf)(Hdf)DabDabPLDabLLDab-NH$_2$ | 1565.03 - 1792.12 | 1590.58 - 1801.13 |
| CMP 1525 | OctA-DabDabF(Hdf)Dab(Hdf)(Hdf)DabDabPLDabLLDab-NH$_2$ | 1641.06 - 1869.16 | 1636.79-1877.43 |
| CMP1526 | OctA-DabDabI(Hdf)Dab(Hdf)(Hdf)DabDabPLDabLLDab-NH$_2$ | 1607.08 - 1835.17 | 1632.86-1842.63 |
| CMP 1527 | OctA-DabDabL(Hdf)Dab(Hdf)(Hdf)DabDabPLDabLLDab-NH$_2$ | 1593.06 - 1821.16 | 1617.86- 1826.71 |
| CMP1528 | OctA-DabDabV(Hdf)Dab(Hdf)(Hdf)DabDabPLDabLLDab-NH$_2$ | 1607.09 - 1835.17 | 1618.70-1845.44 |
| CMP1529 | OctA-DabDab(Hdf)ADab(Hdf)(Hdf)DabDabPLDabLLDab-NH$_2$ | 1565.03 - 1792.12 | 1591.88 - 1801.86 |

13

| Peptide | Sequence | $[M+H]^+$ (calcd.) | $[M+H]^+$ (obsd.) |
|---|---|---|---|
| CMP1530 | OctA-DabDab(Hdf)FDab(Hdf)(Hdf)DabDabPLDabLLDab-NH$_2$ | 1641.06 - 1869.16 | 1656.30-1863.58 |
| CMP1531 | OctA-DabDab(Hdf)IDab(Hdf)(Hdf)DabDabPLDabLLDab-NH$_2$ | 1607.08 - 1835.17 | 1656.31 - 1863.58 |
| CMP1532 | OctA-DabDab(Hdf)LDab(Hdf)(Hdf)DabDabPLDabLLDab-NH$_2$ | 1593.06 - 1821.16 | 1555.92-1830.25 |
| CMP1533 | OctA-DabDab(Hdf)VDab(Hdf)(Hdf)DabDabPLDabLLDab-NH$_2$ | 1607.09 - 1835.17 | 1618.56-1830.68 |
| CMP1534 | OctA-DabDab(Hdf)(Hdf)DabA(Hdf)DabDabPLDabLLDab-NH$_2$ | 1565.03 - 1792.12 | 1621.78 - 1811.35 |
| CMP1535 | OctA-DabDab(Hdf)(Hdf)DabF(Hdf)DabDabPLDabLLDab-NH$_2$ | 1641.06 - 1869.16 | 1673.14-1867.79 |
| CMP1536 | OctA-DabDab(Hdf)(Hdf)DabI(Hdf)DabDabPLDabLLDab-NH$_2$ | 1607.08 - 1835.17 | 1600.98 - 1823.51 |
| CMP1537 | OctA-DabDab(Hdf)(Hdf)DabL(Hdf)DabDabPLDabLLDab-NH$_2$ | 1593.06 - 1821.16 | 1648.17 - 1823.75 |
| CMP1538 | OctA-DabDab(Hdf)(Hdf)DabV(Hdf)DabDabPLDabLLDab-NH$_2$ | 1607.09 - 1835.17 | 1643.14-1858.92 |

| Peptide | Sequence | [M+H]⁺ (calcd.) | [M+H]⁺ (obsd.) |
|---|---|---|---|
| CMP1539 | OctA-DabDab(Hdf)(Hdf)Dab(Hdf)ADabDabPLDabLLDab-NH$_2$ | 1565.03 - 1792.12 | 1594.93 - 1803.68 |
| CMP1540 | OctA-DabDab(Hdf)(Hdf)Dab(Hdf)FDabDabPLDabLLDab-NH$_2$ | 1641.06 - 1869.16 | 1675.67 - 1893.76 |
| CMP1541 | OctA-DabDab(Hdf)(Hdf)Dab(Hdf)IDabDabPLDabLLDab-NH$_2$ | 1607.08 - 1835.17 | 1635.90-1829.84 |
| CMP1542 | OctA-DabDab(Hdf)(Hdf)Dab(Hdf)LDabDabPLDabLLDab-NH$_2$ | 1593.06 - 1821.16 | 1590.73 - 1832.18 |
| CMP1543 | OctA-DabDab(Hdf)(Hdf)Dab(Hdf)VDabDabPLDabLLDab-NH$_2$ | 1607.09 - 1835.17 | 1623.07 - 1847.86 |
| CMP1601 | OctA-DabDabAFDabLADabDabPLDabLLDab-NH$_2$ | 1683.11 | 1682.44 |
| CMP1602 | OctA-DabDabAFDabVADabDabPLDabLLDab-NH$_2$ | 1669.09 | 1668.53 |
| CMP1603 | OctA-DabDabAFDabVVDabDabPLDabLLDab-NH$_2$ | 1697.12 | 1696.54 |
| CMP1604 | OctA-DabDabVFDabVADabDabPLDabLLDab-NH$_2$ | 1697.12 | 1699.56 |
| CMP1701 | OctA-SDabALDabLADabDabPLDabLLDab-NH$_2$ | 1636.09 | 1638.79 |
| CMP1702 | OctA-DabSALDabLADabDabPLDabLLDab-NH$_2$ | 1636.09 | 1638.56 |
| CMP1703 | OctA-DabDabALSLADabDabPLDabLLDab-NH$_2$ | 1636.09 | 1635.86 |
| CMP1704 | OctA-DabDabALDabLASDabPLDabLLDab-NH$_2$ | 1636.09 | 1635.13 |
| CMP1705 | OctA-DabDabALDabLADabSPLDabLLDab-NH$_2$ | 1636.09 | 1635.09 |
| CMP1706 | OctA-DabDabALDabLADabDabPLSLLDab-NH$_2$ | 1636.09 | 1642.02 |
| CMP1707 | OctA-DabDabALDabLADabDabPLDabLLS-NH$_2$ | 1636.09 | 1636.40 |
| CMP1708 | OctA-DabDabALDabLADabDabPLSLLS-NH$_2$ | 1623.06 | 1622.85 |

(continued)

| Peptide | Sequence | [M+H]$^+$ (calcd.) | [M+H]$^+$ (obsd.) |
|---|---|---|---|
| CMP1709 | OctA-DabDabDabAFDabVADabDabPLSLLDab-NH$_2$ | 1655.06 | 1655.98 |

Example 1. Identification of sensitizing effects of CMP1203 and CMP1301 to CMP1314 peptides (MIC assay)

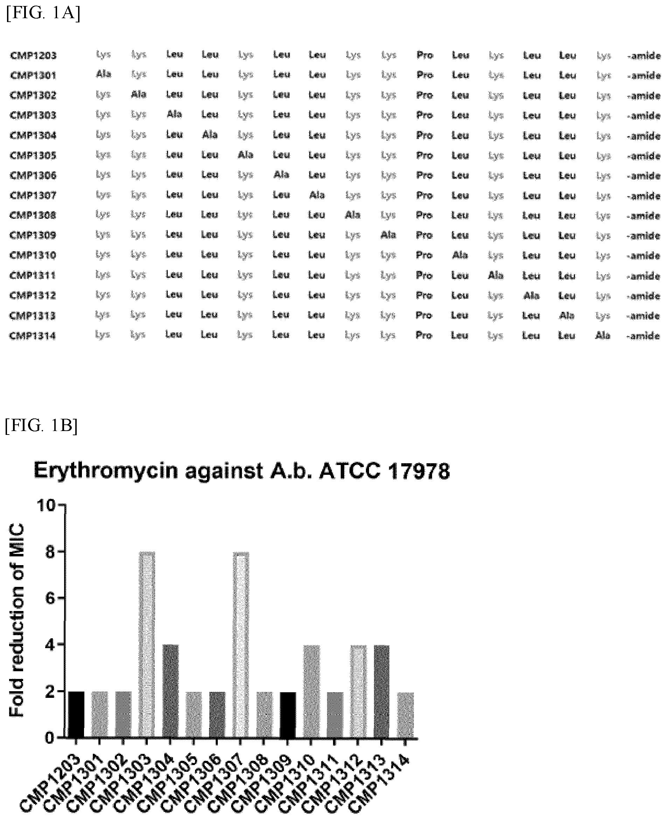

In this example, it was attempted to identify sensitizing effects of CMP1203 and CMP1301 to CMP1314 (FIG. 1A: respective peptides are described).

Each peptide at a concentration of 0.4 uM was used together with the two gram - positive antibiotics erythromycin and novobiocin to identify their effect on *Acinetobacter baumannii* ATCC 17978 (MIC assay). *Acinetobacter baumannii,* which is Gram-negative bacteria commonly classified as an opportunistic pathogen, is a human-infecting bacterium with a high probability of nosocomial infection because it has broad-spectrum antibacterial resistance and is able to survive for a long time even in a low-humidity environment. For each of the gram-positive antibiotics, as compared with MIC of each antibiotic in a case of being used alone, fold reduction of MIC of each antibiotic was measured in a case where each antibiotic was used together with each peptide.

All MIC assay experiments were performed using the Clinical and Laboratory Standards Institute (CLSI) guidelines (Clinical & Laboratory Standards Institute: CLSI Guidelines, 2017). Bacterial cells cultured overnight were diluted to 0.05 McFarland standard using McFarland (KIT DENSICHEK PLUS INSTRUMENT, Biomerieux) in Muller Hinton II Broth (cation-adjusted) (Difco). The cells were inoculated at $1.5 \times 10^5$ CFU per 200 $\mu$L solution of each well in a 96-well plate. The peptide and the antibiotic were prepared using 2-fold serially diluted concentrations. For an insoluble antibiotic, an experiment was conducted using 2.5% (v/v) DMSO under a condition that did not affect bacterial growth. Incubation was performed at 37°C for 18 hours, and then absorbance was measured at 600 nm using an EPOCH2 microplate reader (BioTek, Winooski, USA) to calculate a MIC value. The MIC value was defined as a concentration of the antibiotic or peptide at which bacterial growth was inhibited to a level of lower than 10% growth of the positive control. Fold reduction was calculated using the following expression.

$$\text{Fold reduction} = \text{MIC of antibiotic alone} / \text{MIC of antibiotic in presence of sensitizing peptide}$$

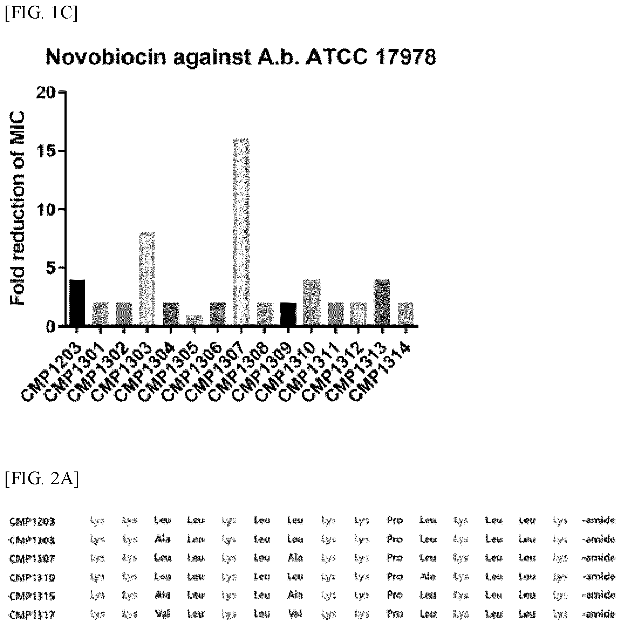

The results are illustrated in FIG. 1.

It was identified that in a case of being used together with erythromycin, all of CMP1203 and CMP1301 to CMP1314 decreased an amount of erythromycin used by two times or higher (FIG. 1B). In particular, as compared with CMP1203, a high sensitizing effect was achieved in a case where leucine was substituted with alanine with lower hydrophobicity (CMP1303, CMP1304, CMP1307, CMP1310, CMP1312, or CMP1313), which resulted in a MIC reduction effect by about 4 to 8 times. In particular, CMP1303 and CMP1307 exhibited a 8 times higher sensitizing effect.

Even for novobiocin, it was identified that all of the peptides of the present disclosure decreased its amount used as compared with a case where novobiocin was used alone (FIG. 1C). In particular, in a case where leucine was substituted with alanine, CMP1303 and CMP1307 exhibited an about 7 to 16 times higher sensitizing effect than CMP1203.

Example 2. Identification of sensitizing effects of peptides CMP1303, CMP1307, CMP1310, CMP1315, andCMP1317 (MIC assay)

In this example, it was attempted to identify a sensitizing effect of each peptide at a concentration of 0.31 uM. CMP1303, CMP1307, CMP1310, CMP1315, and CMP1317 were selected as peptides. In particular, since it was identified in Example 1 that the peptides CMP1301 and CMP1307 exhibited excellent sensitizing ability to the antibacterial agents, erythromycin and novobiocin, against *A. baumannii* ATCC 17978, the peptides CMP1315 and CMP1317 were additionally used in this example to identify their sensitizing effect on fusidic acid.

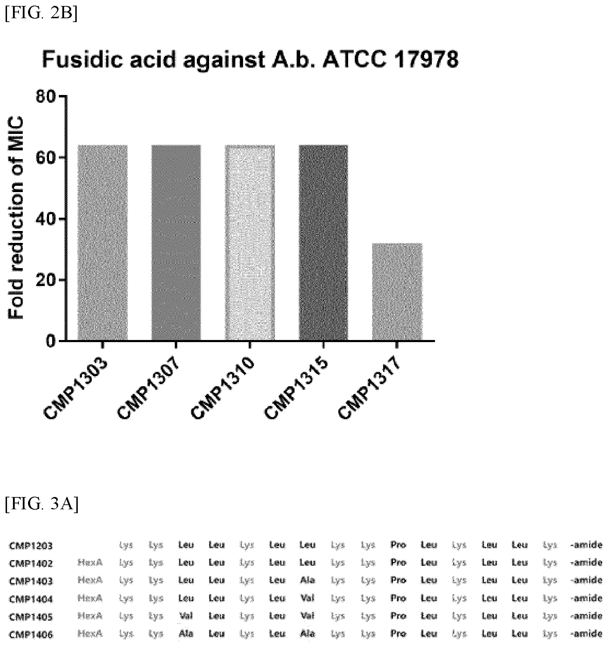

The same MIC assay method as in Example 1 was used except that the peptide concentration was 0.31 $\mu$M. The results are illustrated in FIG. 2.

In a case where the peptide of the present disclosure and fusidic acid were used together, a decreased MIC concentration was observed in all cases as compared with a case where fusidic acid was used alone.

Example 3. Identification of sensitizing effects of peptides lipidated with HexA (MIC assay)

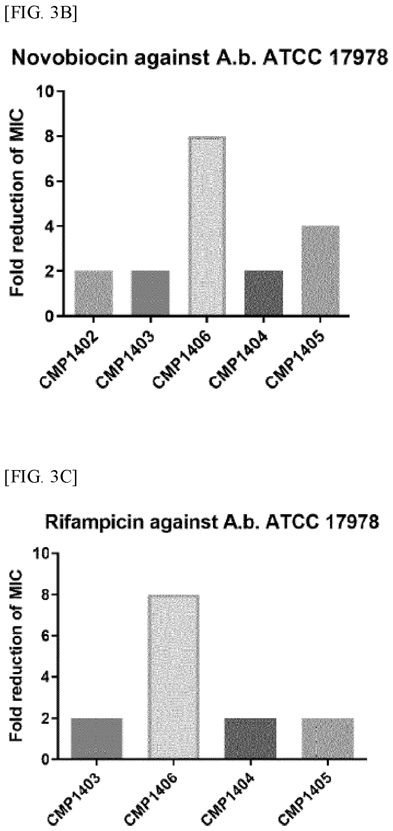

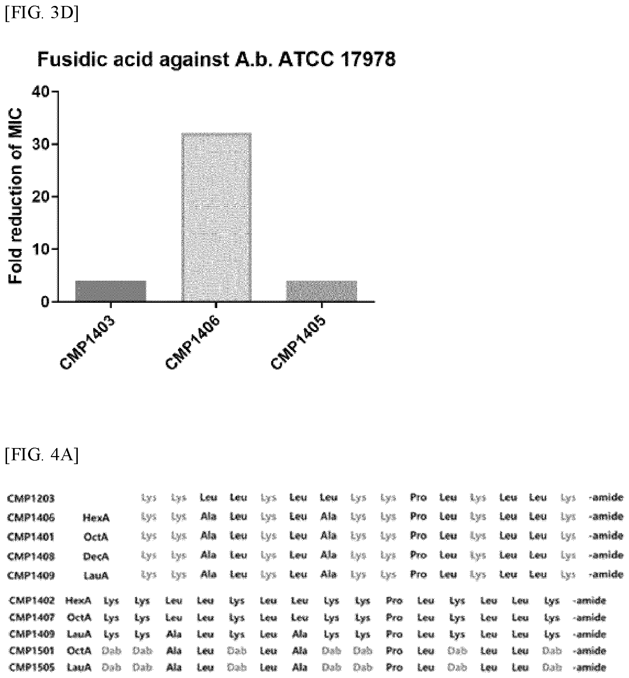

In this example, in a case where peptides whose N-terminus was lipidated with hexanoic acid (HexA) were used together with the three antibacterial agents (novobiocin, rifampicin, or fusidic acid), a sensitizing effect of each peptide was observed. The same MIC assay method as in Example 1 was used except that the peptide concentration was 0.31 $\mu$M. The results are illustrated in FIG. 3.

In a case where each of the peptides of the present disclosure and each of the antibacterial agents were used together, a decreased MIC concentration was observed in all cases as compared with a case where the antibacterial agent was used alone. In particular, for the peptide CMP1406 at a concentration of 0.31 $\mu$M (0.56 $\mu$g/ml), its sensitizing effect was observed to be 8 times stronger for novobiocin, 8 times stronger for rifampicin, and 32 times stronger for fusidic acid.

Example 4. Identification of sensitizing effects of peptides lipidated with various fatty acids (MIC assay)

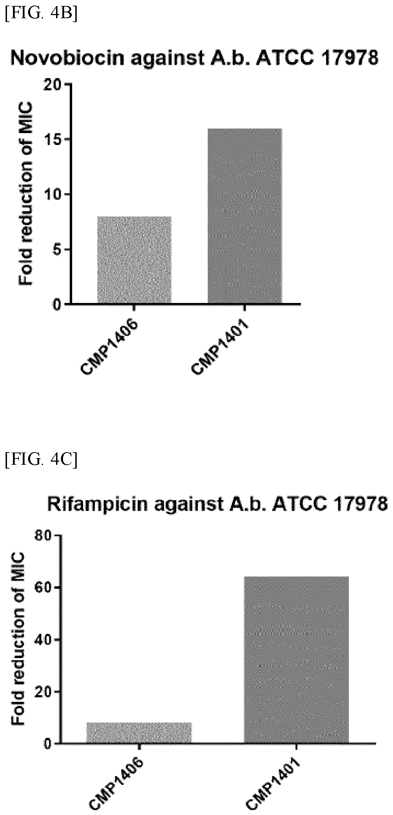

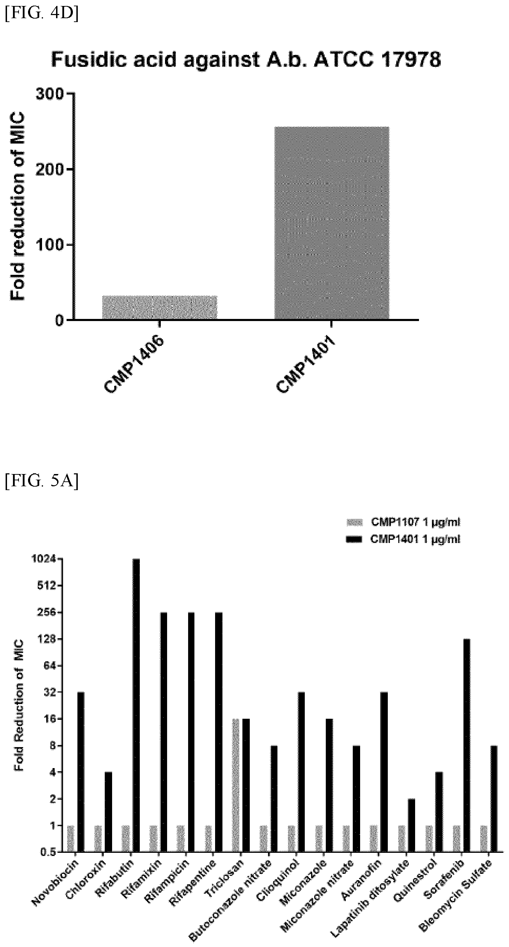

In this example, for peptides whose amino acid sequence was the same as the peptide CMP1406, which was identified in Example 3 to exhibit an excellent sensitizing effect at a low concentration, and whose N-terminus was lipidated with one of fatty acids of various lengths, a sensitizing effect of each peptide was observed. The same MIC assay method as in Example 3 was used. The results are illustrated in FIG. 4.

In a case where each of the peptides of the present disclosure and each of the antibacterial agents were used together, a decreased MIC concentration was observed in all cases as compared with a case where the antibacterial agent was used alone. In particular, for the peptide CMP1401, its sensitizing effect was observed to be 16 times stronger for novobiocin, 64 times stronger for rifampicin, and 250 times stronger for fusidic acid, than a case where the antibacterial agent was used alone.

Example 5. Comparison of sensitizing effect between known sensitizing peptide and CMP1401 orCMP1501 (MIC assay)

Using the same MIC assay method as in Example 1, sensitizing effects of the peptide L9P (CMP1107, 1 ug/ml), which is known as a conventional sensitive AMP, and CMP1401 (1 ug/ml) were compared on existing antibacterial agents against *E. coli* ATCC 25922 (FIG. 5A).

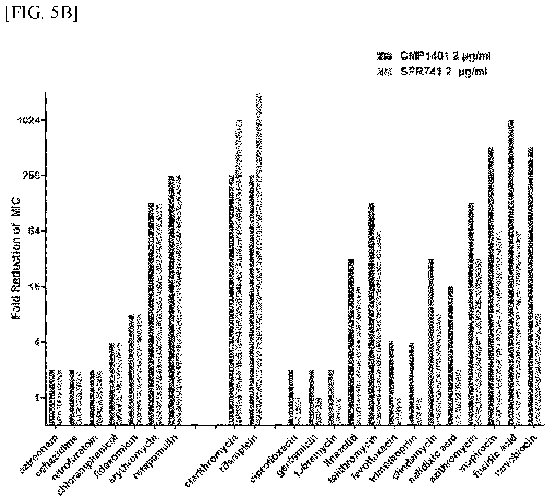

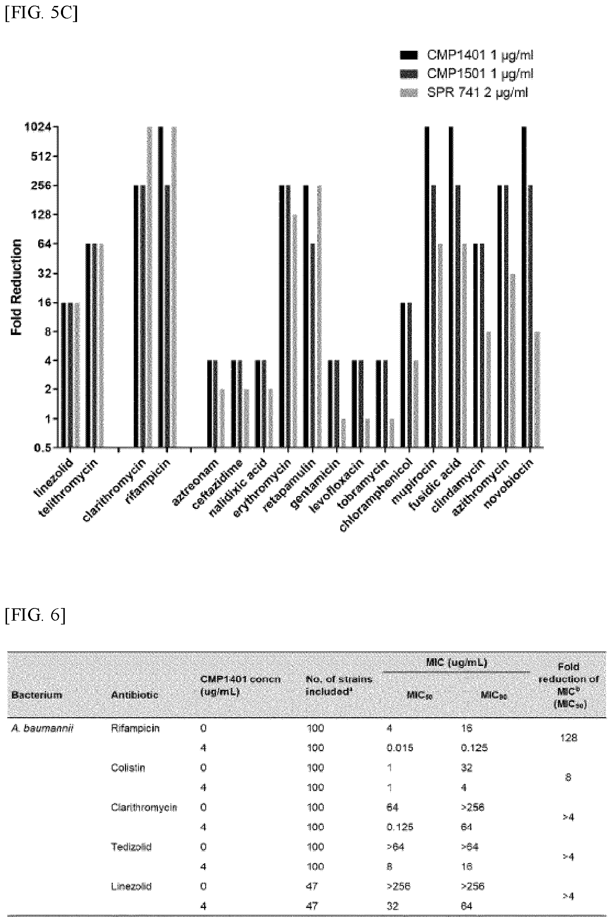

In addition, MIC screening was used to compare sensitizing effects of the competitive drug SPR741 and the peptide CMP1401 or CMP1501 on the existing antibacterial agents against *E. coli* ATCC 25922 (FIGS. 5B and 5C). SPR741, which is a previously known polymyxin B (PMB) analog, is a sensitive AMP, and is a drug that has been identified through preclinical experiments to be effective against Gram-negative bacteria in a case of being used together with existing Gram-positive antibiotics.

As a result, the peptide CMP1401 of the present disclosure exhibited a very good sensitizing effect as compared with the conventionally known peptide L9P (FIG. 5A). Furthermore, it was identified that even in a case of being compared with SPR741, the peptide CMP1401 or CMP1501 of the present disclosure exhibited almost the same or much better sensitizing effect (FIGS. 5B and 5C). In particular, it was identified that even in a case where a concentration of CMP1401 or CMP1501 was decreased by half as compared with SRP741, each peptide exhibited an equal or superior effect (FIG. 5C).

Example 6. Sensitizing effect of CMP1401 in clinical strain ($MIC_{50}$ and $MIC_{90}$)

In the clinical strain *A. baumannii,* $MIC_{50}$ and $MIC_{90}$ values were obtained in a case where the existing antibacterial agent rifampicin, colistin, clarithromycin, tedizolid, or linezolid was used alone or in combination with CMP1401 at 4 ug/ml. 100 *A. baumannii* clinical strains were used for each antibacterial agent group, in which 50 carbapenem-resistant strains and 50 carbapenem-susceptible strains were selected.

The results are illustrated in FIG. 6. The "Fold reduction of $MIC_{90}$" in FIG. 6 indicates a $MIC_{90}$ value observed in a case where the antibacterial agent is used in combination with CMP1401 as compared with a case where the antibacterial agent is used alone.

From the results, it can be seen that the peptide CMP1401 of the present disclosure exhibited a sensitizing effect of at least 4 times, and at most 128 times, as compared with a case where the antibacterial agent was used alone.

Example 7. Sensitizing effects of peptides CMP1501, and CMP1601 to CMP1604 (MIC assay)

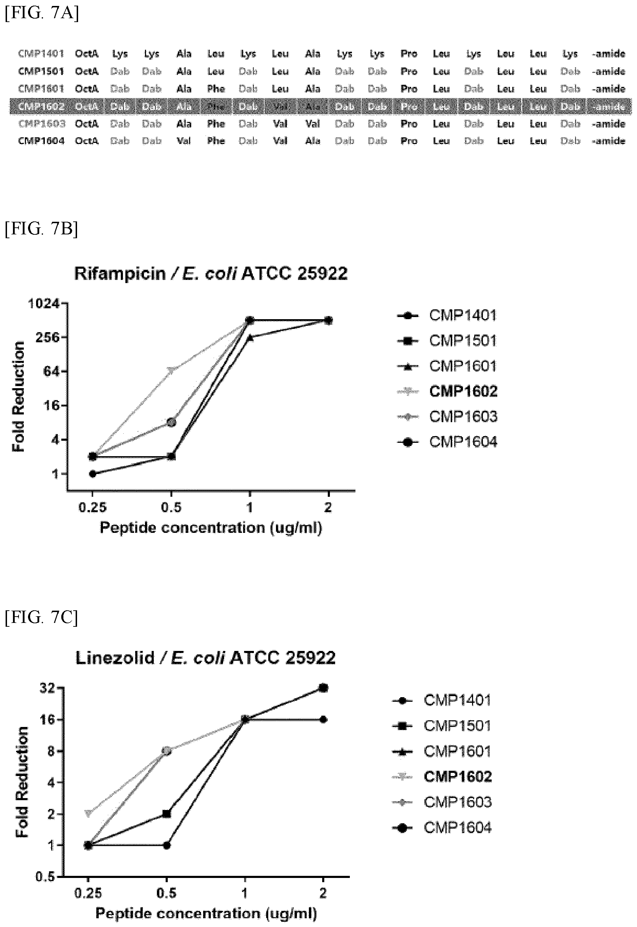

Using the same MIC assay method as in Example 1, sensitizing effects of CMP1501, and CMP1601 to CMP1604 were identified on rifampicin and linezolid against *E. coli* ATCC 25922 (FIG. 7).

As a result, CMP1501, and CMP1601 to CMP1604 exhibited excellent sensitizing effects even at a low concentration of 0.5 ug/ml.

Example 8. Sensitizing effects of peptides CMP1701 to CMP1709 (MIC assay)

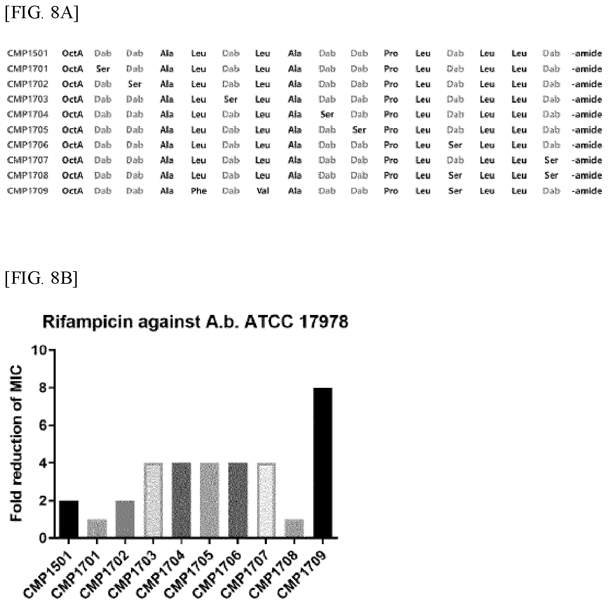

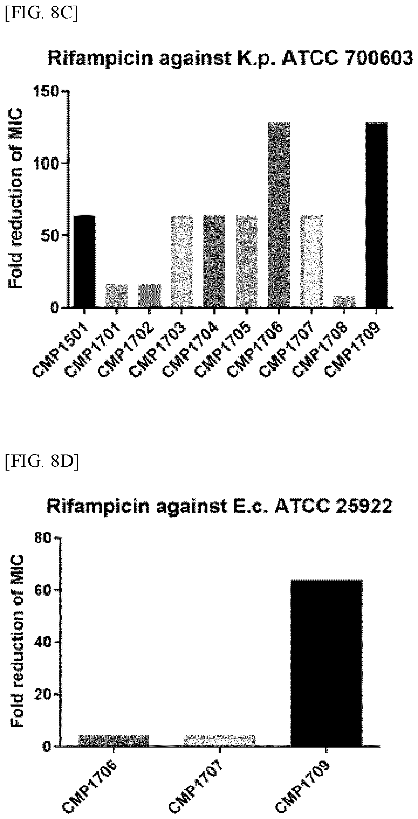

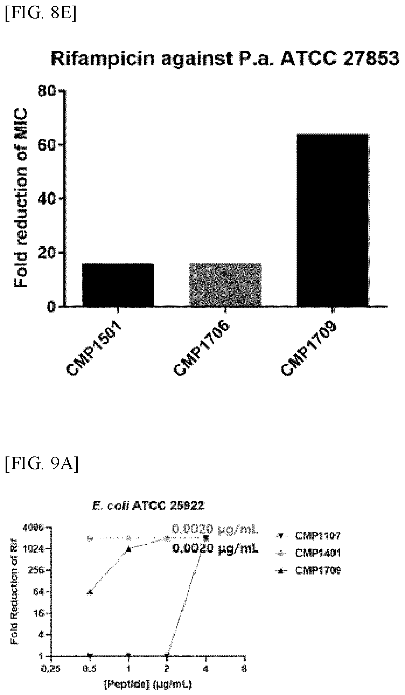

Using the same MIC assay method as in Example 1, sensitizing effects of CMP1701 to CMP1709 were identified on rifampicin against four Gram-negative strains (A.b. ATCC 17978, K.p. ATCC 700603, E.c. ATCC 25922, and P.a. ATCC 27853) (FIG. 8). The peptides were used at a concentration of 0.5 ug/mL for A.b. ATCC 17978, K.p. ATCC 700603, and E.c. ATCC 25922, and used at a concentration of 1.0 ug/mL for P.a. ATCC 27853.

As a result, it was identified that all of the peptides exhibited an excellent sensitizing effect at low concentrations. In particular, CMP1709 exhibited an about 8 to 120 times higher sensitizing effect than a case where rifampicin was used alone, against 4 types of Gram-negative bacteria.

Example 9. Comparison of sensitizing effects of known sensitizing peptide and CMP1401 or CMP1709 (MIC assay)

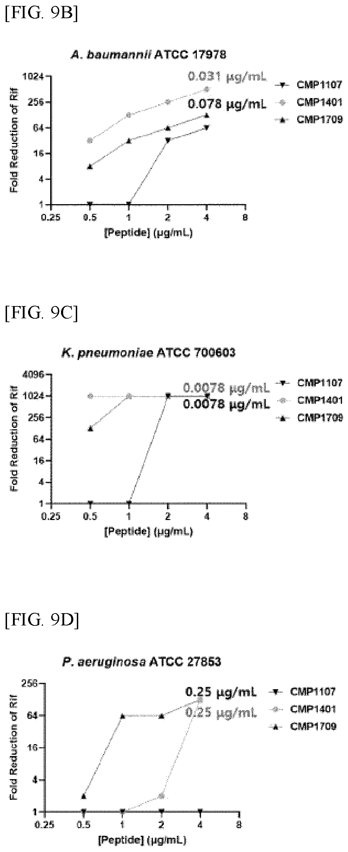

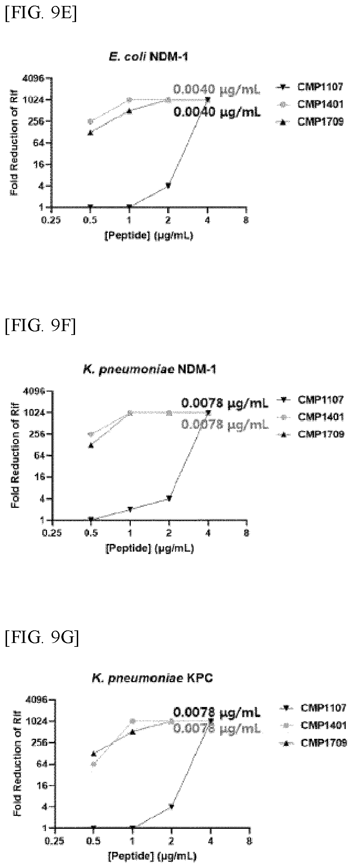

Using the same MIC assay method as in Example 1, sensitizing effects of the existing sensitizing peptide AMP L9P (CMP1107) and CMP1401 or CMP1709 were compared on rifampicin or colistin against the four Gram-negative strains E.c. ATCC 25922, A.b. ATCC 17978, K.p. ATCC 700603, and P.a. ATCC 27853, or CRE (E.c. NDM-1, K.p. NDM-1, or K.p. KPC) (FIGS. 9 and 10).

As a result, the peptide CMP1401 or CMP1709 of the present disclosure exhibited a very good sensitizing effect as compared with the conventionally known peptide L9P.

Example 10. Sensitizing effect of CMP1709 in clinical strains ($MIC_{50}$ and $MIC_{90}$)

In a case where the existing antibacterial agent rifampicin or colistin was used alone and a case where the existing antibacterial agent rifampicin or colistin was used in combination with CMP1709 at 4 ug/ml, $MIC_{50}$ and $MIC_{90}$ values were obtained against the clinical strains *E. coli, K. pneumoniae, P. aeruginosa, E. cloacae, C. freundii,* and *S. marcescens*. The selected clinical strains *E. coli, K. pneumoniae, E. cloacae, C. freundii,* and *S. marcescens* were all carbapenem-resistant strains, and the selected clinical strain *P. aeruginosa* included 43 strains of imipenem- andmeropenem-resistant strains, and 60 strains of imipenem- andmeropenem-sensitive strains.

The results are illustrated in FIG. 11 (results not shown for colistin). The "Fold reduction of $MIC_{90}$" in FIG. 11 indicates a $MIC_{90}$ value observed in a case where the antibacterial agent was used in combination with CMP1709 as compared with a case where the antibacterial agent was used alone. From the results, it can be seen that the peptide CMP1709 of the present disclosure exhibited a sensitizing effect of at least 2 times, and at most 1024 times, as compared with a case where the antibacterial agent was used alone. Even in a case where colistin was used, the peptide exhibited a two times higher sensitizing effect against the strains *E. coli* and *E. cloacae* (results not shown in FIG. 11).

Example 11. Identification of membrane-destroying ability of peptides (enzyme assay)

It was observed, through an enzyme assay, whether the sensitizing peptide destroyed the outer and inner membranes of Gram-negative bacteria. In the presence of the sensitizing peptide, it was observed whether in a case where 1.5 mM ONPG was used together therewith, β-galactosidase was released from *E. coli ATCC* 25922 for the inner membrane, and whether in a case where 200 μM CENTA was used together therewith, β-lactamase was released from *E. coli* NDM-1 for the outer membrane.

Specifically, to determine leakage of the outer membrane, activity of the β-lactamase released from the outer membrane was evaluated. New Delhi metallo-β-lactamase-1 (NDm-1)-producing *E. coli* cells were washed three times with DPBS and suspended in the same buffer. The cells were diluted to an OD600 of 0.2 and treated with the peptide at a concentration of 0.63 or 1.30 uM at 37°C for 1 h. After centrifugation, the supernatant was incubated with 200 μM CENTATM β-lactamase substrate at 37°C for 1 hour. An optical density was obtained by measuring absorbance of a product of enzyme reaction at 405 nm using a microplate reader (Molecular Devices Co., Menlo Park, CA).

To determine leakage of the inner membrane, release of the cytoplasmic enzyme β-galactosidase was evaluated. The bacteria were diluted to OD600 = 1 and 1.5 mM ONPG (O-nitrophenyl-β-D-galactopyranoside) was added. Absorbance of a product of enzyme reaction was measured at 420 nm. The other procedures were carried out in the same manner as in the experiment for outer membrane destruction.

The results are illustrated in FIG. 12. The y-axis is absorbance measured at 405 nm, and higher absorbance is observed as a higher amount of β-lactamase is released due to destruction of the membrane.

It was observed that melittin, which is known to have a mechanism of action by which both the inner and outer membranes of bacteria are destroyed, destroyed both the inner and outer membranes of bacteria. On the other hand, it was identified that CMP1407 and CMP1401 did not destroy these membranes at an almost equivalent level as compared with L9P (CMP1107), which is a previously known sensitizing peptide and is known to not destroy the membranes of Gram-negative bacteria.

Example 12. Toxicity analysis of peptides (hemolysis assay)

To identify toxicity of the peptides, human red blood cell (hRBC) hemolytic activity assay (hemolysis assay) was performed.

Specifically, percent hemolysis of human red blood cells (hRBC) was determined by comparing the cells incubated in 100% deionized water (DW) as a positive control and peptide-free PBS as a negative control. hRBCs were washed 3 times with PBS using centrifugation at 500 g for 5 min, and 5% (v/v) hematocrit was suspended in PBS buffer (Hyclone). The peptide was diluted using 2-fold serial dilution with PBS, and hRBCs were added at $2.5 \times 10^8$ cells to each well. The sample was incubated at 37°C for 4 hours. After centrifugation at 1400 rpm for 5 minutes, 180 $\mu$L of the supernatant was transferred to a transparent flat-bottom 96-well plate. An amount of hemoglobin released by the peptide was determined by measuring absorbance at 405 nm using an EPOCH2 microplate reader (BioTek, Winooski, USA).

$$\text{Hemolytic activity (\%)} = (\text{absorbance of sample - absorbance of PBS}) /$$

$$(\text{absorbance of 100\% DW - A405 of PBS}) \times 100$$

The results are illustrated in FIG. 13. Unlike melittin that exhibited hemolysis of hRBCs starting from low concentrations, the peptides of the present disclosure exhibited a very insignificant hemolysis level except for CMP1407.

Example 13. Toxicity assay of peptides (WST-1 assay)

In this example, toxicity of the peptide was identified by measuring host cell viability through WST-1 assay of HeLa cells or HK-2 cells as host cells.

Specifically, culture was performed for HeLa cells in DMEM and HK-2 cells in RPMI, at a condition of 37°C with 5% $CO_2$. The cells were respectively cultured in a cell culture plate, and then removed with trypsin. The cells were seeded respectively at $1 \times 10^4$ cells and $7 \times 10^3$ cells per well in a 96-well plate. After 24 hours, the peptide diluted in 2-fold dilution was added to the media, and then culture was performed again at a condition of 37°C with 5% $CO_2$ for 24 hours. 10 uL of WST-1 reagent was added per well, and reaction was allowed to proceed for 30 minutes at a condition of 37°C with 5% $CO_2$. Then, UV absorbance was measured at 450/700 nm with a 96-well plate reader.

The results are illustrated in FIG. 14. All of the peptides of the present disclosure exhibited high cell viability even at high concentrations.

Example 14. *In vivo* efficacy analysis of combination therapy of rifampicin and CMP1401 (*in vivo* test of CMP1401)

Using a mouse model, *in vivo* efficacy of the peptide CMP1401 and rifampicin (Rif) was analyzed in a case where they are used as monotherapy and combination therapy.

14.1 Experiments using neutropenic mouse pneumoniae mouse model (immunosuppressed mouse *A. baumannii* 801 pneumonia infection survival model)

To induce neutropenia in 6-week-old female ICR(CD-1) mice weighing 23 g to 27 g (Orient Bio, Seongnam, Korea), the mice were treated (subcutaneously injected) with 150 mg/kg and 100 mg/kg of cyclophosphamide (CP), respectively, 4 days and 1 day before pneumonia infection. For a mouse model for survival analysis, the mice were infected intranasally with $1 \times 10^8$ CFU *A. baumannii* 801; and for a mouse model for colony number counting, the mice were infected intranasally with $1 \times 10^6$ CFU *A. baumannii* 801.

Survival analysis

An effect of co-administration of rifampicin and the peptide CMP1401 was identified in a neutropenic mouse pneumoniae mouse model.

Rifampicin was dissolved in a carrier (10% TWEEN80, 5% DMSO, and 85% saline) and used. Rifampicin was administered by subcutaneous injection twice daily for 2 days (2.5 mg/kg BID). The sensitizing peptide CMP1401 of the present disclosure was administered by intraperitoneal injection 4 times daily for 2 days (25 mg/kg QID). Six mice were used per each group. The Mantel-Cox test was used to obtain p values of the group in which co-administration was performed and the group in which rifampicin was administered alone.

As a result, as illustrated in FIG. 15A, the group, in which 2.5 mg/kg of rifampicin was administered alone, exhibited a survival rate of about 16.7%, and the group, in which 2.5 mg/kg of rifampicin and 25 mg/kg of the peptide CMP1401 were co-administered, exhibited a survival rate of 50% which was about 33.3% higher than the group in which rifampicin was administered alone. The group, in which the peptide CMP1401 was administered alone, did not exhibit any antibacterial activity because all mice died.

Measurement of number of colonies

In addition to the survival rate in the neutropenic mouse pneumoniae mouse model, the number of colonies of *A. baumannii* 801 was measured in the mouse lungs. The mice were euthanized and lungs were harvested in sterile saline. The lungs were homogenized and serially diluted in sterile saline. The homogenized lungs were applied to tryptic soy agar plates, incubated at 37°C overnight, and the number of colonies was counted. The two-sidedMan Whitney U-testwas used to obtain p values of the group in which co-administration was performed and the group in which rifampicin was administered alone (*: $p < 0.05$, **: $p < 0.005$). Six mice were used per each group.

As a result, as illustrated in FIG. 15B, the group, in which 2.5 mg/kg of rifampicin and 25 mg/kg of the peptide CMP1401 were co-administered, exhibitedthe number of colonies that was about 12 times fewer than the group in which 2.5 mg/kg of rifampicin was administered alone. In addition, the group, in which rifampicin and CMP1401 were co-administered, exhibited the number of colonies that was about 60 times fewer than the untreated group (control). The group, in which rifampicin and CMP1401 were co-administered, exhibited the number of colonies that was about 30 times fewer than the group in which rifampicin and SPR741 were co-administered.

14.2 Experiments using neutropenic mouse thigh model

To induce neutropenia in 6-week-old female ICR (CD-1) mice weighing 23 g to 27 g (Orient Bio, Seongnam, Korea), the mice were treated (subcutaneously injected) with 150 mg/kg and 100 mg/kg of cyclophosphamide (CP), respectively, 5 days and 2 days before thigh infection. *A. baumannii* ATCC 17978 was harvested, washed, and suspended in sterile saline. The mice were anesthetized and infected with $1 \times 10^5$ CFU *A. baumannii* ATCC 17978 in the right and left thighs. 20 mg/kg of rifampicin was administered by subcutaneous injection 1 and 5 hours after infection. The sensitizing peptide CMP1401 was administered by intraperitoneal injection 1, 3, 5, and 7 hours after infection. The mice were euthanized 9 hours after bacterial injection. Both thighs were harvested and suspended in sterile saline. The thighs were homogenized and serially diluted in sterile saline. The homogenized thighs were applied to tryptic soy agar plates, incubated overnight at 37°C, and the number of colonies was counted. The two-sided Man Whitney U-test was used to obtain p values of the group in which co-administration was performed and the group in which rifampicin was administered alone (*: $p < 0.05$).

The results are illustrated in FIG. 15C. In the neutropenic mouse thigh model infected with *A. baumannii* ATCC 17978, the combination therapy of rifampicin and the peptide CMP1401 exhibited excellent therapeutic efficacy. In a case where the peptide CMP1401 was administered at 100 mg/kg in the presence of 20 mg/kg of rifampicin, the number of colonies was decreased 14-fold as compared with a case where rifampicin was administered alone. In addition, changes in the number of colonies were observed in a case where a dose of the peptide CMP1401 was changed in the group in which co-administration was performed in the presence of 20 mg/kg of rifampicin. No changes were observed in the number of colonies in a case where the peptide CMP1401 was administered alone at 50 mg/kg, indicating that the peptide CMP1401 enhances antibacterial ability of rifampicin rather than exhibiting its own antibacterial activity.

Example 15. *In vivo* efficacy analysis of rifampicin or colistin plus CMP1401 (*in vivo* test of CMP1401)

In this example, it was attempted to identify an effect of co-administration with rifampicin or colistin in an immunosuppressed rat *A. baumannii* 801 pneumonia infection model.

The same colony number counting test method as in Example 14.1 was used except that three SD male rats were used per group instead of the mice, and infected with *A. baumannii* 801 through the airways followed by administration of colistin or rifampicin via subcutaneous injection and administration of CMP1401 via intravenous infusion for 1 hour. The results are illustrated in FIG. 16.

32 mg/kg of colistin is known as a clinical dose used for treatment of *A. baumannii* 801. Therefore, an effect observed in a case where 32 mg/kg of colistin was used alone may be a criterion for comparing effectiveness of co-administration of CMP1401 and an antibacterial agent in this example.

According to the results of FIG. 16, it was identified that in a case where rifampicin and CMP1401 were co-administered, in particular, in a case where 5 mg/kg of rifampicin and 3 mg/kg or 6 mg/kg of CMP1401 were co-administered, an effect was obtained which is comparable to that observed in a case where 32 mg/kg of colistin was administered. Accordingly, it was identified that the peptide of the present disclosure exhibited a clinically effective effect in a case of being used together with rifampicin.

Example 16. Analysis of *in vivo* efficacy of combination therapy ofrifampicin and CMP1709 (*in vivo* test of CMP1709)

Using a neutropenic mouse thigh model (immunosuppressed mouse *E. coli* NDM-1 thigh infection model), *in vivo* efficacy of the peptide CMP1401 and rifampicin (Rif) was analyzed in a case where they are used as monotherapy and combination therapy. Experiments were performed in the same manner as in Example 14.2.

The results are illustrated in FIG. 17. Combination therapy of rifampicin and the peptide CMP1709 exhibited excellent therapeutic efficacy in a neutropenic mouse thigh model infected with *E. coli NDM-1.* In a case where the peptide CMP1709 was administered at 75 mg/kg in the presence of 20 mg/kg of rifampicin, the number of colonies was decreased 8.7-fold as compared with a case where rifampicin was administered alone. In addition, changes in the number of colonies were observed in a case where a dose of the peptide CMP1709 was changed in the group in which co-administration was performed in the presence of 20 mg/kg of rifampicin. No changes were observed in the number of colonies in a case where the peptide CMP1709 was administered alone, indicating that the peptide CMP1709 enhances antibacterial ability of rifampicin rather than exhibiting its own antibacterial activity.

## Claims

1. A peptide or peptide analog represented by Formula 1 :

    <Formula 1> $\quad X_1X_2X_3X_4X_5X_6X_7X_8X_9X_{10}X_{11}X_{12}X_{13}X_{14}X_{15}$

    in the formula,
    $X_1$, $X_2$, $X_5$, $X_8$, $X_9$, $X_{12}$, and $X_{15}$ are each independently a hydrophilic amino acid or non-proteinogenic amino acid, provided that at least one of them is Ala or Ser,
    $X_3$, $X_4$, $X_6$, $X_7$, $X_{11}$, $X_{13}$, and $X_{14}$ are each independently a hydrophobic amino acid or a mixture thereof,
    $X_{10}$ is Pro,
    a $C_6$ to $C_{16}$ fatty acid is bound to any one position of $X_1$ to $X_{15}$, and
    $X_1$ is N-terminus and $X_{15}$ is C-terminus.

2. The peptide or peptide analog of claim 1, wherein $X_1$, $X_2$, $X_5$, $X_8$, $X_9$, $X_{12}$, and $X_{15}$ are each independently a hydrophilic amino acid selected from Lys, Arg, His, and derivatives thereof, or 2,3-diaminopropionic acid (Dap), 2,4-diaminobutanoic acid (Dab), or ornithine (Orn), provided that at least one of them is Ala or Ser; and $X_3$, $X_4$, $X_6$, $X_7$, $X_{11}$, $X_{13}$, and $X_{14}$ are each independently a hydrophobic amino acid selected from Leu, Ala, Ile, Phe, Val, Trp, or Tyr, or Hdf, wherein Hdf is a mixture of amino acids comprising Leu, Ala, Val, Ile, and Phe in equal amounts.

3. The peptide or peptide analog of claim 2, wherein $X_1$, $X_2$, $X_5$, $X_8$, $X_9$, $X_{12}$, and $X_{15}$ are each independently Lys,Ala, Ser, or 2,4-diaminobutanoic acid (Dab).

4. The peptide or peptide analog of claim 2, wherein $X_3$, $X_4$, $X_6$, and $X_7$ are each independently Leu, Ala, Val, Ile, Phe, or Hdf.

5. The peptide or peptide analog of claim 2, wherein $X_{11}$, $X_{13}$, and $X_{14}$ are each independently Leu or Ala.

6. The peptide or peptide analog of claim 2, wherein a $C_6$ to $C_{12}$ fatty acid is bound to a position of $X_1$.

7. The peptide or peptide analog of claim 2, wherein $X_1$, $X_2$, $X_5$, $X_8$, $X_9$, $X_{12}$, and $X_{15}$ are each independently Lys, and $X_3$, $X_4$, $X_6$, $X_7$, $X_{11}$, $X_{13}$, and $X_{14}$ are each independently Leu.

8. The peptide or peptide analog of claim 2, wherein $X_1$, $X_2$, $X_5$, $X_8$, $X_9$, $X_{12}$, and $X_{15}$ are each independently Lys or Ala, and $X_3$, $X_4$, $X_6$, $X_7$, $X_{11}$, $X_{13}$, and $X_{14}$ are each independently Leu, Ala or Val, provided that at least one of $X_1$ to $X_{15}$ except for $X_{10}$ is Ala or Val.

9. The peptide or peptide analog of claim 2, wherein $X_1$, $X_2$, $X_5$, $X_8$, $X_9$, $X_{12}$, and $X_{15}$ are each independently Lys; $X_3$, $X_7$, and $X_{11}$ are each independently Leu, Ala, or Val; $X_4$, $X_6$, $X_{13}$, and $X_{14}$ are each independently Leu; and a $C_6$ to $C_{12}$ fatty acid is bound to a position of $X_1$.

10. The peptide or peptide analog of claim 2, wherein $X_1$, $X_2$, $X_5$, $X_8$, $X_9$, $X_{12}$, and $X_{15}$ are each independently Dab; $X_3$, $X_4$, $X_6$, and $X_7$ are each independently Leu, Ala, Ile, Phe, Val, or Hdf, wherein at least two of $X_3$, $X_4$, $X_6$, and $X_7$ are each independently Ala, Ile, Phe, Val, or Hdf; $X_{11}$, $X_{13}$, and $X_{14}$ are each independently Leu; and a $C_8$ or $C_{12}$ fatty acid is bonded to a position of $X_1$.

11. The peptide or peptide analog of claim 2, wherein $X_1$, $X_2$, $X_5$, $X_8$, $X_9$, $X_{12}$, and $X_{15}$ are each independently Dab; $X_3$,

$X_4$, $X_6$, and $X_7$ are each independently Leu, Ala, Phe, or Val, wherein at leastthree of $X_3$, $X_4$, $X_6$, and $X_7$ are each independently Ala, Phe, or Val; $X_{11}$, $X_{13}$, and $X_{14}$ are each independently Leu; and a $C_8$ fatty acid is bound to a position of $X_1$.

**12.** The peptide or peptide analog of claim 2, wherein $X_1$, $X_2$, $X_5$, $X_8$, $X_9$, $X_{12}$, and $X_{15}$ are each independently Dab or Ser, wherein at least one of $X_1$, $X_2$, $X_5$, $X_8$, $X_9$, $X_{12}$, and $X_{15}$ is each independently Ser; $X_3$ and $X_7$ are each independently Ala; $X_4$ and $X_6$ are each independently Leu, Phe or Val; $X_{11}$, $X_{13}$, and $X_{14}$ are each independently Leu; and a $C_8$ fatty acid is bound to a position of $X_1$.

**13.** The peptide or peptide analog of claim 2, wherein $X_1$, $X_2$, $X_5$, $X_8$, $X_9$, $X_{12}$, and $X_{15}$ are each independently Lys, Ala, Ser, or Dab; $X_3$, $X_4$, $X_6$, and $X_7$ are each independently Leu, Ala, Val, Ile, Phe, or Hdf; $X_{10}$ is Pro; $X_{11}$, $X_{13}$, and $X_{14}$ are each independently Leu or Ala; and a $C_6$ to $C_{12}$ fatty acid is bound to a position of $X_1$.

**14.** A peptide or peptide analog, comprising the amino acid sequence of any one of SEQ ID NOs: 1 to 67.

**15.** An antibacterial pharmaceutical composition, comprising the peptide or peptide analog of any one of claims 1 to 14 and a drug.

**16.** The antibacterial pharmaceutical composition of claim 15, wherein the pharmaceutical composition exhibits antibacterial activity against Gram-negative bacteria.

**17.** The antibacterial pharmaceutical composition of claim 16, wherein the Gram-negative bacteria is at least one selected from the group consisting of E. *coli, Acinetobacter baumannii, Klebsiella pneumoniae, Pseudomonas aeruginosa, Enterobacter cloacae, Citrobacter freundii,* and *Serratia marcescens.*

**18.** The antibacterial pharmaceutical composition of claim 17, wherein the drug is erythromycin, novobiocin, fusidic acid, rifampicin, rifaximin, chloroxine, gatifloxacin, lomefloxacin, rifabutin, rifapentine, daptomycin, nisin, tigecycline, aztreonam, ceftazidime, nitrofurantoin, chloramphenicol, fidaxomicin, retapamulin, cefepime, mecillinam, meropenem, vancomycin, clarithromycin, fosfomycin, ramoplanin, ciprofloxacin, gentamycin, tobramycin, linezolid, telithromycin, levofloxacin, trimethoprim, clindamycin, nalidixic acid, azithromycin, mupirocin, daptomycin, linezolid, nitrofurantoin, fidaxomicin, aztreonam, retapamulin, tobramycin, tedizolid, albamycin, auranofin, capitrol, triclosan, butoconazole, miconazole, clioquinol, lapatinib, sorafenib, bleomycin, quinestrol, aztreonam, or colistin.

**19.** The antibacterial pharmaceutical composition of claim 15, wherein the peptide or peptide analog and the drug are administered simultaneously as one formulation, or administered simultaneously or sequentially as separate formulations.

**20.** A conjugate comprising the peptide or peptide analog of any one of claims 1 to 14 with a drug linked thereto.

[FIG. 1A]

| | | | | | | | | | | | | | | | |
|---|---|---|---|---|---|---|---|---|---|---|---|---|---|---|---|
| CMP1203 | Lys | Lys | Leu | Leu | Lys | Leu | Leu | Lys | Lys | Pro | Leu | Lys | Leu | Leu | Lys | -amide |
| CMP1301 | Ala | Lys | Leu | Leu | Lys | Leu | Leu | Lys | Lys | Pro | Leu | Lys | Leu | Leu | Lys | -amide |
| CMP1302 | Lys | Ala | Leu | Leu | Lys | Leu | Leu | Lys | Lys | Pro | Leu | Lys | Leu | Leu | Lys | -amide |
| CMP1303 | Lys | Lys | Ala | Leu | Lys | Leu | Leu | Lys | Lys | Pro | Leu | Lys | Leu | Leu | Lys | -amide |
| CMP1304 | Lys | Lys | Leu | Ala | Lys | Leu | Leu | Lys | Lys | Pro | Leu | Lys | Leu | Leu | Lys | -amide |
| CMP1305 | Lys | Lys | Leu | Leu | Ala | Leu | Leu | Lys | Lys | Pro | Leu | Lys | Leu | Leu | Lys | -amide |
| CMP1306 | Lys | Lys | Leu | Leu | Lys | Ala | Leu | Lys | Lys | Pro | Leu | Lys | Leu | Leu | Lys | -amide |
| CMP1307 | Lys | Lys | Leu | Leu | Lys | Leu | Ala | Lys | Lys | Pro | Leu | Lys | Leu | Leu | Lys | -amide |
| CMP1308 | Lys | Lys | Leu | Leu | Lys | Leu | Leu | Ala | Lys | Pro | Leu | Lys | Leu | Leu | Lys | -amide |
| CMP1309 | Lys | Lys | Leu | Leu | Lys | Leu | Leu | Lys | Ala | Pro | Leu | Lys | Leu | Leu | Lys | -amide |
| CMP1310 | Lys | Lys | Leu | Leu | Lys | Leu | Leu | Lys | Lys | Pro | Ala | Lys | Leu | Leu | Lys | -amide |
| CMP1311 | Lys | Lys | Leu | Leu | Lys | Leu | Leu | Lys | Lys | Pro | Leu | Ala | Leu | Leu | Lys | -amide |
| CMP1312 | Lys | Lys | Leu | Leu | Lys | Leu | Leu | Lys | Lys | Pro | Leu | Lys | Ala | Leu | Lys | -amide |
| CMP1313 | Lys | Lys | Leu | Leu | Lys | Leu | Leu | Lys | Lys | Pro | Leu | Lys | Leu | Ala | Lys | -amide |
| CMP1314 | Lys | Lys | Leu | Leu | Lys | Leu | Leu | Lys | Lys | Pro | Leu | Lys | Leu | Leu | Ala | -amide |

[FIG. 1B]

**Erythromycin against A.b. ATCC 17978**

[FIG. 1C]

Novobiocin against A.b. ATCC 17978

[FIG. 2A]

| CMP1203 | Lys | Lys | Leu | Leu | Lys | Leu | Leu | Lys | Lys | Pro | Leu | Lys | Leu | Leu | Lys | -amide |
|---------|-----|-----|-----|-----|-----|-----|-----|-----|-----|-----|-----|-----|-----|-----|-----|--------|
| CMP1303 | Lys | Lys | Ala | Leu | Lys | Leu | Leu | Lys | Lys | Pro | Leu | Lys | Leu | Leu | Lys | -amide |
| CMP1307 | Lys | Lys | Leu | Leu | Lys | Leu | Ala | Lys | Lys | Pro | Leu | Lys | Leu | Leu | Lys | -amide |
| CMP1310 | Lys | Lys | Leu | Leu | Lys | Leu | Leu | Lys | Lys | Pro | Ala | Lys | Leu | Leu | Lys | -amide |
| CMP1315 | Lys | Lys | Ala | Leu | Lys | Leu | Ala | Lys | Lys | Pro | Leu | Lys | Leu | Leu | Lys | -amide |
| CMP1317 | Lys | Lys | Val | Leu | Lys | Leu | Val | Lys | Lys | Pro | Leu | Lys | Leu | Leu | Lys | -amide |

25

[FIG. 2B]

## Fusidic acid against A.b. ATCC 17978

[FIG. 3A]

| CMP1203 | | Lys | Lys | Leu | Leu | Lys | Leu | Leu | Lys | Lys | Pro | Leu | Lys | Leu | Leu | Lys | -amide |
|---------|------|-----|-----|-----|-----|-----|-----|-----|-----|-----|-----|-----|-----|-----|-----|-----|--------|
| CMP1402 | HexA | Lys | Lys | Leu | Leu | Lys | Leu | Leu | Lys | Lys | Pro | Leu | Lys | Leu | Leu | Lys | -amide |
| CMP1403 | HexA | Lys | Lys | Leu | Leu | Lys | Leu | Ala | Lys | Lys | Pro | Leu | Lys | Leu | Leu | Lys | -amide |
| CMP1404 | HexA | Lys | Lys | Leu | Leu | Lys | Leu | Val | Lys | Lys | Pro | Leu | Lys | Leu | Leu | Lys | -amide |
| CMP1405 | HexA | Lys | Lys | Val | Leu | Lys | Leu | Val | Lys | Lys | Pro | Leu | Lys | Leu | Leu | Lys | -amide |
| CMP1406 | HexA | Lys | Lys | Ala | Leu | Lys | Leu | Ala | Lys | Lys | Pro | Leu | Lys | Leu | Leu | Lys | -amide |

[FIG. 3B]

**Novobiocin against A.b. ATCC 17978**

[FIG. 3C]

**Rifampicin against A.b. ATCC 17978**

[FIG. 3D]

## Fusidic acid against A.b. ATCC 17978

[FIG. 4A]

| CMP1203 | | Lys | Lys | Leu | Leu | Lys | Leu | Leu | Lys | Lys | Pro | Leu | Lys | Leu | Leu | Lys | -amide |
|---|---|---|---|---|---|---|---|---|---|---|---|---|---|---|---|---|---|
| CMP1406 | HexA | Lys | Lys | Ala | Leu | Lys | Leu | Ala | Lys | Lys | Pro | Leu | Lys | Leu | Leu | Lys | -amide |
| CMP1401 | OctA | Lys | Lys | Ala | Leu | Lys | Leu | Ala | Lys | Lys | Pro | Leu | Lys | Leu | Leu | Lys | -amide |
| CMP1408 | DecA | Lys | Lys | Ala | Leu | Lys | Leu | Ala | Lys | Lys | Pro | Leu | Lys | Leu | Leu | Lys | -amide |
| CMP1409 | LauA | Lys | Lys | Ala | Leu | Lys | Leu | Ala | Lys | Lys | Pro | Leu | Lys | Leu | Leu | Lys | -amide |
| | | | | | | | | | | | | | | | | | |
| CMP1402 | HexA | Lys | Lys | Leu | Leu | Lys | Leu | Leu | Lys | Lys | Pro | Leu | Lys | Leu | Leu | Lys | -amide |
| CMP1407 | OctA | Lys | Lys | Leu | Leu | Lys | Leu | Leu | Lys | Lys | Pro | Leu | Lys | Leu | Leu | Lys | -amide |
| CMP1409 | LauA | Lys | Lys | Ala | Leu | Lys | Leu | Ala | Lys | Lys | Pro | Leu | Lys | Leu | Leu | Lys | -amide |
| CMP1501 | OctA | Dab | Dab | Ala | Leu | Dab | Leu | Ala | Dab | Dab | Pro | Leu | Dab | Leu | Leu | Dab | -amide |
| CMP1505 | LauA | Dab | Dab | Ala | Leu | Dab | Leu | Ala | Dab | Dab | Pro | Leu | Dab | Leu | Leu | Dab | -amide |

[FIG. 4B]

**Novobiocin against A.b. ATCC 17978**

[FIG. 4C]

**Rifampicin against A.b. ATCC 17978**

[FIG. 4D]

Fusidic acid against A.b. ATCC 17978

[FIG. 5A]

[FIG. 5B]

[FIG. 5C]

[FIG. 6]

| Bacterium | Antibiotic | CMP1401 concn (ug/mL) | No. of strains included[a] | MIC (ug/mL) | | Fold reduction of MIC[b] (MIC50) |
|---|---|---|---|---|---|---|
| | | | | MIC50 | MIC80 | |
| A. baumannii | Rifampicin | 0 | 100 | 4 | 16 | 128 |
| | | 4 | 100 | 0.015 | 0.125 | |
| | Colistin | 0 | 100 | 1 | 32 | 8 |
| | | 4 | 100 | 1 | 4 | |
| | Clarithromycin | 0 | 100 | 64 | >256 | >4 |
| | | 4 | 100 | 0.125 | 64 | |
| | Tedizolid | 0 | 100 | >64 | >64 | >4 |
| | | 4 | 100 | 8 | 16 | |
| | Linezolid | 0 | 47 | >256 | >256 | >4 |
| | | 4 | 47 | 32 | 64 | |

[FIG. 7A]

| CMP1401 | OctA | Lys | Lys | Ala | Leu | Lys | Leu | Ala | Lys | Lys | Pro | Leu | Lys | Leu | Leu | Lys | -amide |
| CMP1501 | OctA | Dab | Dab | Ala | Leu | Dab | Leu | Ala | Dab | Dab | Pro | Leu | Dab | Leu | Leu | Dab | -amide |
| CMP1601 | OctA | Dab | Dab | Ala | Phe | Dab | Leu | Ala | Dab | Dab | Pro | Leu | Dab | Leu | Leu | Dab | -amide |
| CMP1602 | OctA | Dab | Dab | Ala | Phe | Dab | Val | Ala | Dab | Dab | Pro | Leu | Dab | Leu | Leu | Dab | -amide |
| CMP1603 | OctA | Dab | Dab | Ala | Phe | Dab | Val | Val | Dab | Dab | Pro | Leu | Dab | Leu | Leu | Dab | -amide |
| CMP1604 | OctA | Dab | Dab | Val | Phe | Dab | Val | Ala | Dab | Dab | Pro | Leu | Dab | Leu | Leu | Dab | -amide |

[FIG. 7B]

Rifampicin / *E. coli* ATCC 25922

[FIG. 7C]

Linezolid / *E. coli* ATCC 25922

[FIG. 8A]

| | | | | | | | | | | | | | | | | | |
|---|---|---|---|---|---|---|---|---|---|---|---|---|---|---|---|---|---|
| CMP1501 | OctA | Dab | Dab | Ala | Leu | Dab | Leu | Ala | Dab | Dab | Pro | Leu | Dab | Leu | Leu | Dab | -amide |
| CMP1701 | OctA | Ser | Dab | Ala | Leu | Dab | Leu | Ala | Dab | Dab | Pro | Leu | Dab | Leu | Leu | Dab | -amide |
| CMP1702 | OctA | Dab | Ser | Ala | Leu | Dab | Leu | Ala | Dab | Dab | Pro | Leu | Dab | Leu | Leu | Dab | -amide |
| CMP1703 | OctA | Dab | Dab | Ala | Leu | Ser | Leu | Ala | Dab | Dab | Pro | Leu | Dab | Leu | Leu | Dab | -amide |
| CMP1704 | OctA | Dab | Dab | Ala | Leu | Dab | Leu | Ala | Ser | Dab | Pro | Leu | Dab | Leu | Leu | Dab | -amide |
| CMP1705 | OctA | Dab | Dab | Ala | Leu | Dab | Leu | Ala | Dab | Ser | Pro | Leu | Dab | Leu | Leu | Dab | -amide |
| CMP1706 | OctA | Dab | Dab | Ala | Leu | Dab | Leu | Ala | Dab | Dab | Pro | Leu | Ser | Leu | Leu | Dab | -amide |
| CMP1707 | OctA | Dab | Dab | Ala | Leu | Dab | Leu | Ala | Dab | Dab | Pro | Leu | Dab | Leu | Leu | Ser | -amide |
| CMP1708 | OctA | Dab | Dab | Ala | Leu | Dab | Leu | Ala | Dab | Dab | Pro | Leu | Ser | Leu | Leu | Ser | -amide |
| CMP1709 | OctA | Dab | Dab | Ala | Phe | Dab | Val | Ala | Dab | Dab | Pro | Leu | Ser | Leu | Leu | Dab | -amide |

[FIG. 8B]

Rifampicin against A.b. ATCC 17978

[FIG. 8C]

Rifampicin against K.p. ATCC 700603

[FIG. 8D]

Rifampicin against E.c. ATCC 25922

[FIG. 8E]

Rifampicin against P.a. ATCC 27853

[FIG. 9A]

E. coli ATCC 25922

[FIG. 9B]

*A. baumannii* ATCC 17978

[FIG. 9C]

*K. pneumoniae* ATCC 700603

[FIG. 9D]

*P. aeruginosa* ATCC 27853

[FIG. 9E]

**E. coli NDM-1**

[FIG. 9F]

**K. pneumoniae NDM-1**

[FIG. 9G]

**K. pneumoniae KPC**

[FIG. 10A]

*E. coli* ATCC 25922

[FIG. 10B]

*P. aeruginosa* ATCC 27853

[FIG. 10C]

*K. pneumoniae* KPC

[FIG. 11]

| Bacterium | Antibiotic | CMP1709 concn (ug/mL) | No. of strains included[a] | MIC (ug/mL) | | Fold reduction of MIC[b] (MIC$_{90}$) |
|---|---|---|---|---|---|---|
| | | | | MIC$_{50}$ | MIC$_{90}$ | |
| E. coli | Rifampicin | 0 | 40 | 16 | 32 | 512 |
| | | 4 | 40 | 0.015 | 0.06 | |
| K. pneumoniae | Rifampicin | 0 | 147 | 32 | 32 | 256 |
| | | 4 | 147 | 0.015 | 0.125 | |
| P. aeruginosa | Rifampicin | 0 | 103 | 32 | >64 | >8 |
| | | 4 | 103 | 0.25 | 8 | |
| E. cloacae | Rifampicin | 0 | 20 | 32 | 32 | 512 |
| | | 4 | 20 | 0.015 | 0.06 | |
| C. freundii | Rifampicin | 0 | 20 | >32 | >32 | 1024 |
| | | 4 | 20 | 0.008 | 0.03 | |
| S. marcescens | Rifampicin | 0 | 8 | 32 | >32 | >2 |
| | | 4 | 8 | 2 | 16 | |

[FIG. 12A]

Outer Membrane

[FIG. 12B]

[FIG. 13A]

[FIG. 13B]

**Hemolysis assay**

- MELITTIN
- CMP1203
- CMP1315
- CMP1406
- CMP1401
- CMP1402
- CMP1407
- CMP1408
- CMP1409
- CMP1505
- CMP1501
- CMP1709

[FIG. 14A]

**HeLa cell WST-1 assay**

- CMP1203
- CMP1407
- CMP1401

[FIG. 14B]

HK-2 Cell WST-1 assay

[FIG. 15A]

A.b. 801 mouse pneumonia infection model

[FIG. 15B]

A.b. 801 mouse pneumonia infectoin model

[FIG. 15C]

A.b. ATCC 17978 mouse thigh infection model

[FIG. 16]

A.b. 801 Rat pneumonia infection model

[FIG. 17]

E.c. NDM-1 mouse thigh infection model

[FIG. 18]

| | N- | 1 | 2 | 3 | 4 | 5 | 6 | 7 | 8 | 9 | 10 | 11 | 12 | 13 | 14 | 15 | -C |
|---|---|---|---|---|---|---|---|---|---|---|---|---|---|---|---|---|---|
| CMP1524 | OctA- | Dab | Dab | Ala | Hdf | Dab | Hdf | Hdf | Dab | Dab | Pro | Leu | Dab | Leu | Leu | Dab | -amide |
| CMP1525 | OctA- | Dab | Dab | Phe | Hdf | Dab | Hdf | Hdf | Dab | Dab | Pro | Leu | Dab | Leu | Leu | Dab | -amide |
| CMP1526 | OctA- | Dab | Dab | Ile | Hdf | Dab | Hdf | Hdf | Dab | Dab | Pro | Leu | Dab | Leu | Leu | Dab | -amide |
| CMP1527 | OctA- | Dab | Dab | Leu | Hdf | Dab | Hdf | Hdf | Dab | Dab | Pro | Leu | Dab | Leu | Leu | Dab | -amide |
| CMP1528 | OctA- | Dab | Dab | Val | Hdf | Dab | Hdf | Hdf | Dab | Dab | Pro | Leu | Dab | Leu | Leu | Dab | -amide |
| CMP1529 | OctA- | Dab | Dab | Hdf | Ala | Dab | Hdf | Hdf | Dab | Dab | Pro | Leu | Dab | Leu | Leu | Dab | -amide |
| CMP1530 | OctA- | Dab | Dab | Hdf | Phe | Dab | Hdf | Hdf | Dab | Dab | Pro | Leu | Dab | Leu | Leu | Dab | -amide |
| CMP1531 | OctA- | Dab | Dab | Hdf | Ile | Dab | Hdf | Hdf | Dab | Dab | Pro | Leu | Dab | Leu | Leu | Dab | -amide |
| CMP1532 | OctA- | Dab | Dab | Hdf | Leu | Dab | Hdf | Hdf | Dab | Dab | Pro | Leu | Dab | Leu | Leu | Dab | -amide |
| CMP1533 | OctA- | Dab | Dab | Hdf | Val | Dab | Hdf | Hdf | Dab | Dab | Pro | Leu | Dab | Leu | Leu | Dab | -amide |
| CMP1534 | OctA- | Dab | Dab | Hdf | Hdf | Dab | Ala | Hdf | Dab | Dab | Pro | Leu | Dab | Leu | Leu | Dab | -amide |
| CMP1535 | OctA- | Dab | Dab | Hdf | Hdf | Dab | Phe | Hdf | Dab | Dab | Pro | Leu | Dab | Leu | Leu | Dab | -amide |
| CMP1536 | OctA- | Dab | Dab | Hdf | Hdf | Dab | Ile | Hdf | Dab | Dab | Pro | Leu | Dab | Leu | Leu | Dab | -amide |
| CMP1537 | OctA- | Dab | Dab | Hdf | Hdf | Dab | Leu | Hdf | Dab | Dab | Pro | Leu | Dab | Leu | Leu | Dab | -amide |
| CMP1538 | OctA- | Dab | Dab | Hdf | Hdf | Dab | Val | Hdf | Dab | Dab | Pro | Leu | Dab | Leu | Leu | Dab | -amide |
| CMP1539 | OctA- | Dab | Dab | Hdf | Hdf | Dab | Hdf | Ala | Dab | Dab | Pro | Leu | Dab | Leu | Leu | Dab | -amide |
| CMP1540 | OctA- | Dab | Dab | Hdf | Hdf | Dab | Hdf | Phe | Dab | Dab | Pro | Leu | Dab | Leu | Leu | Dab | -amide |
| CMP1541 | OctA- | Dab | Dab | Hdf | Hdf | Dab | Hdf | Ile | Dab | Dab | Pro | Leu | Dab | Leu | Leu | Dab | -amide |
| CMP1542 | OctA- | Dab | Dab | Hdf | Hdf | Dab | Hdf | Leu | Dab | Dab | Pro | Leu | Dab | Leu | Leu | Dab | -amide |
| CMP1543 | OctA- | Dab | Dab | Hdf | Hdf | Dab | Hdf | Val | Dab | Dab | Pro | Leu | Dab | Leu | Leu | Dab | -amide |

## INTERNATIONAL SEARCH REPORT

| International application No. |
|---|
| **PCT/KR2021/004232** |

| **A.** | **CLASSIFICATION OF SUBJECT MATTER** |
|---|---|

**A61K 38/00**(2006.01)i; **A61K 38/08**(2006.01)i; **A61P 31/04**(2006.01)i

According to International Patent Classification (IPC) or to both national classification and IPC

| **B.** | **FIELDS SEARCHED** |
|---|---|

Minimum documentation searched (classification system followed by classification symbols)

A61K 38/00(2006.01); A23K 20/147(2016.01); A23L 33/18(2016.01); A61K 31/421(2006.01); A61K 47/62(2017.01); A61P 31/04(2006.01); C07K 7/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 그람 음성균(Gram-Negative Bacteria), 항균 펩타이드(antimicrobial peptides), 친수성(hydrophilicity), 소수성(hydrophobicity), 지방산(fatty acid)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X<br><br>Y | KR 10-2017-0033790 A (SEOUL NATIONAL UNIVERSITY R&DB FOUNDATION) 27 March 2017 (2017-03-27)<br>     See abstract; claims 1-5, 20-21 and 23; tables 1-2 and 13-15; and figures 15-19. | 14<br><br>1-13,15-20 |
| Y | US 2019-0216939 A1 (SI CHUAN UNIVERSITY) 18 July 2019 (2019-07-18)<br>     See abstract; claims 1-9; and paragraph [0010]. | 1-13,15-20 |
| X<br>Y | 최윤화. 프롤린으로 꺾인 양면성 알파나선구조 펩타이드의 그람 음성균 외막 섭동에 의한 다양한 항생제와의 시너지 항균효과 향상에 관한 연구. 서울대학교 대학원 교육학석사학위논문. December 2016, pp. 1-45 (CHOI, Yunhwa. Proline hinged amphipathic α-helical peptide enhances synergistic antimicrobial activity with various antibiotics by perturbing outer membrane of gram-negative bacteria. Master's thesis, Department of Science Education, The Graduate School Seoul National University.).<br>     See abstract; pages 6-12; tables 1-3; and figure 1. | 14<br>1-13,15-20 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 July 2021** | **06 July 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2021/004232**

### C. DOCUMENTS CONSIDERED TO BE RELEVANT

| Category* | Citation of document, with indication, where appropriate, of the relevant passages | Relevant to claim No. |
|---|---|---|
| A | KR 10-2003-0061718 A (ANYGEN CO., LTD.) 22 July 2003 (2003-07-22)<br>See entire document. | 1-20 |
| A | KR 10-2018-0056226 A (INDUSTRY-ACADEMIC COOPERATION FOUNDATION, KUNSAN NATIONAL UNIVERSITY) 28 May 2018 (2018-05-28)<br>See entire document. | 1-20 |

Form PCT/ISA/210 (second sheet) (July 2019)

| INTERNATIONAL SEARCH REPORT | International application No. |
|---|---|
| | **PCT/KR2021/004232** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/KR2021/004232**

| Patent document cited in search report | | | Publication date (day/month/year) | Patent family member(s) | | | Publication date (day/month/year) |
|---|---|---|---|---|---|---|---|
| KR | 10-2017-0033790 | A | 27 March 2017 | CN | 109415411 | A | 01 March 2019 |
| | | | | EP | 3351553 | A1 | 25 July 2018 |
| | | | | JP | 2018-529689 | A | 11 October 2018 |
| | | | | JP | 2020-128386 | A | 27 August 2020 |
| | | | | KR | 10-1811437 | B1 | 25 January 2018 |
| | | | | US | 2018-0339016 | A1 | 29 November 2018 |
| | | | | WO | 2017-048092 | A1 | 23 March 2017 |
| US | 2019-0216939 | A1 | 18 July 2019 | CN | 107441501 | A | 08 December 2017 |
| | | | | CN | 107441501 | B | 10 November 2020 |
| | | | | CN | 107446019 | A | 08 December 2017 |
| | | | | EP | 3480208 | A1 | 08 May 2019 |
| | | | | JP | 2019-527044 | A | 26 September 2019 |
| | | | | WO | 2018-001383 | A1 | 04 January 2018 |
| KR | 10-2003-0061718 | A | 22 July 2003 | None | | | |
| KR | 10-2018-0056226 | A | 28 May 2018 | KR | 10-1899552 | B1 | 17 September 2018 |
| | | | | US | 10077293 | B2 | 18 September 2018 |
| | | | | US | 2018-0141984 | A1 | 24 May 2018 |
| | | | | WO | 2018-092955 | A1 | 24 May 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1811437

**Non-patent literature cited in the description**

- *Clinical & Laboratory Standards Institute: CLSI Guidelines,* 2017